# EUROPEAN PATENT APPLICATION

(11) **EP 4 011 862 A1**
(43) Date of publication of application: **15.06.2022**
(21) Application number: 20849738.8
(22) Date of filing: 05.08.2020
(51) Int. Cl.: C07C 237/30, A61K 31/136, A61K 8/42, A61P 43/00, A61P 11/00

(54) **NOVEL COMPOUND FOR INHIBITING HISTONE ACETYLTRANSFERASE P300 AND ANTI-FIBROSIS COMPOSITION COMPRISING SAME**

(30) Priority: 05.08.2019 KR 20190095153
(71) Applicant: Refure Life Science Inc., Seoul 03722 (KR)
(72) Inventor: YOON, Ho Geun, Goyang-si Gyeonggi-do 10450 (KR); SOHN, Myung Hyun, Seoul 06288 (KR); PARK, Soo Yeon, Seongnam-si Gyeonggi-do 13469 (KR); HONG, Jung Yeon, Goyang-si Gyeonggi-do 10446 (KR); LEE, Soo Yeon, Seoul 06695 (KR); KWON, Youngjoo, Seoul 03709 (KR); NA, Younghwa, Seoul 03709 (KR); HWANG, Soo-Yeon, Seoul 04129 (KR); SHIN, Jaeho, Chilgok-gun Gyeongsangbuk-do 39813 (KR)
(74) Representative: Müller, Christian Stefan Gerd
(86) International application number: PCT/KR2020/010303
(87) International publication number: WO 2021/025447

(57) **Abstract**

The present invention relates to a novel compound which enables additional hydrogen bonding with a specific amino acid position of histone acetyltransferase (HAT) p300 through the structural analysis of the HAT p300. The novel compound of the present invention has an excellent inhibitory effect on HAT p300 activity, and thus may be very effectively used to prevent, ameliorate or treat fibrosis that is a disease associated with the activation of HAT p300.

## Description

### [Technical Field]

The present invention relates to a novel compound for inhibiting histone acetyltransferase p300; an antifibrotic composition including the novel compound; and various uses thereof.

### [Background Art]

Tissue is bound to the extracellular matrix, and includes a population of highly organized cells surrounded by the vascular network. Fibrosis or fiberization is a process of abnormal accumulation of collagen matrices caused by injury or inflammation which causes structural and functional changes in various tissues. In the case of fibrosis, the excessive accumulation of fibrous connective tissues (e.g., collagen matrix) replacing the normal tissues falls within most etiologic factors, regardless of the site of fibrosis occurrence. Progressive fibrosis in the kidneys, liver, fat, lung, heart, bone or bone marrow, skin, and the like is a major cause of death or pain.

Especially, among the types of fibrosis, pulmonary fibrosis that is fibrosis that develops in the lungs refers to a disease that induces tissue fiberization to cause a severe structural change in lung tissue while allowing chronic inflammatory cells to infiltrate into the alveolar wall of the lung tissue. When fiberization proceeds due to any cause of fibrosis, the lung tissue becomes hard, and the alveolar wall thickens to reduce an amount of supply of oxygen through blood, which makes it difficult for patients to breathe. In recent years, in the field of medicine, there are no treatment methods in which it is possible to completely recover the lung tissue whose fiberization is already advanced. Thus, the symptom of fibrosis develops unless the fibrosis is found at an early stage of progression or without any lung transplantation, and eventually results in patients' death in 3 to 5 years.

Methods of treating pulmonary fibrosis found at the early stage of fibrosis progression includes a treatment method using steroid-based drugs such as steroids, azathioprine, cyclophosphamide; a treatment method using antioxidants such as acetylcysteine; and a treatment method involving the administration of growth factors such as cytokines, interferon-γ (IFN-γ); and the like. Although the treatment methods using the steroid-based drugs and the antioxidants have been consistently studied and reported since the year 2000, there are no drugs whose efficacy is clearly proven so far. Currently available drugs are reported to cause systemic side effects when administered for a long time or cause side effects such as tolerance, and the like. As a therapeutic method that is recently receiving much attention, the treatment method involving the administration of growth factors is a relatively fundamental therapeutic approach using "interferon" which suppresses the production of transforming growth factor-β (TGF-β) known as an important factor for pulmonary fibrosis. Because this therapeutic approach is based on the analysis of the cause of this disease, the approach has fewer side effects and superior efficacy, compared to the treatment methods using the steroid-based drugs or the antioxidants, and thus various types of treatment methods using injections, aerosols, etc. have been reported. However, because the exact cause of pulmonary fibrosis is unknown so far, single-dose components (such as interferon, etc.) may have a temporary therapeutic effect and may be effective only in some patients. Therefore, there is a need for continuous research and clinical trials.

As a mechanism to regulate the functions of numerous proteins, including transcription factors that play a direct role in transcription, depending on this need, research on components associated with the post-translational modification of proteins, such as acetylation of histone proteins, which have an influence on phenomena such as expression of the proteins, and the like under the control of a lysine residue in amino acids, which are basic units that constitute a protein, has been conducted. However, there is still insufficient research on substances having excellent effects.

### [Disclosure]

### [Technical Problem]

The present invention is directed to providing a novel compound capable of inhibiting histone acetyltransferase (HAT) p300.

The present invention is also directed to providing a composition for preventing, ameliorating or treating a disease associated with the HAT p300, which includes the novel compound that inhibits the HAT p300.

The present invention is also directed to providing a method of preventing, ameliorating or treating a disease associated with the HAT p300 using the novel compound that inhibits the HAT p300.

However, technical problems to be solved in the present invention are not limited to the above-described problems, and other problems which are not described herein will be fully understood by those of ordinary skill in the art from the following descriptions.

### [Technical Solution]

According to one embodiment of the present invention, there is provided a compound selected from a compound represented by the following Formula 1, and a pharmaceutically acceptable salt, an optical isomer, a hydrate, and a solvate thereof: 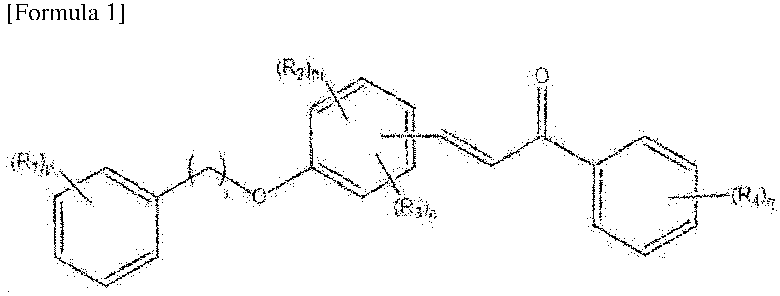 wherein:
p and q are each independently an integer ranging from 1 to 5;
r is an integer ranging from 1 to 4;
m and n are each independently an integer ranging from 1 to 3, provided that m + n is not greater than 4;
R₁ is a carbamoyl group (-C( = O)(NH₂)) or a halogen, wherein when R₁ is present in a plural number, they are the same or different from each other;
R₂ and R₃ are each independently a C₁-C₆ alkoxy group or a halogen, wherein when each of R₂ and R₃ is present in a plural number, they are the same or different from each other; and
R₄ includes any one selected from the group consisting of a C₁-C₆ alkoxy group, an amine group (-NH₂), a carbamoyl group, and a hydroxyl group (-OH), wherein when R₄ is present in a plural number, they are the same or different from each other.

Unless otherwise mentioned in the present invention, the term "halogen" refers to fluorine, chlorine, bromine, or iodine, unless otherwise mentioned.

Unless otherwise mentioned in the present invention, the term "alkoxy group" refers to an alkyl group bound to oxygen, and may, for example, include a methoxy group, an ethoxy group, and the like, but the present invention is not limited thereto.

In the present invention, the term "alkyl" refers to a linear or branched, saturated monovalent hydrocarbon radical. In this case, the alkyl may be optionally substituted with one or more substituents as described in the present invention. Examples of the alkyl may include methyl, ethyl, propyl (including all types of isomers thereof), n-propyl, isopropyl, butyl (including all types of isomers thereof), n-butyl, isobutyl, sec-butyl, t-butyl, pentyl (including all types of isomers thereof), and hexyl (including all types of isomers thereof), but the present invention is not limited thereto.

In the present invention, the "pharmaceutically acceptable salt" should have low toxicity to the human body and not have an adverse effect on the biological activity and physicochemical properties of a parent compound. A pharmaceutically acceptable free acid and an acid addition salt of the basic compound of Formula 1 may be used as the pharmaceutically acceptable salt, but the present invention is not limited thereto.

Types of the preferred salt of the compound according to the present invention may include salts with an inorganic acid or an organic acid. In this case, hydrochloric acid, sulfuric acid, nitric acid, phosphoric acid, perchloric acid, bromic acid, and the like may be used as the inorganic acid. Also, acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, and the like may be used as the organic acid. Organic bases that may be used to prepare an organic base addition salt include tris(hydroxymethyl)methylamine, dicyclohexylamine, and the like. Amino acids that may be used to prepare an amino acid addition salt include natural amino acids such as alanine, glycine, and the like. It will be apparent to a person having ordinary skill in the art that other acids and bases may be used in addition to the exemplified inorganic acids, organic acids, organic bases, and amino acids.

The salt of the present invention may be prepared using a conventional method. For example, the salt of the present invention may be prepared by dissolving the above-described compound of Formula 1 in a solvent that may be mixed with water, such as methanol, ethanol, acetone, 1,4-dioxane, and the like, adding a free acid or a free base thereto, and crystallizing the resulting mixture.

In the present invention, because the "optical isomer" may have an asymmetric carbon center, all types of the optical isomers and mixtures that may be obtained from the compound of the present invention as R or S isomers or racemic compounds fall within the scope of the present invention.

In the present invention, the compound may be represented by the following Formula 2: 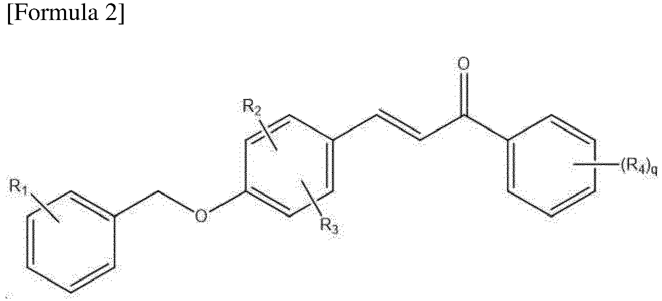 wherein:
each of R₁, R₂, R₃, R₄, and q are as defined above in Formula 1.

According to one preferred embodiment of the present invention, p and q may be each independently an integer ranging from 1 to 3. Preferably, p may be an integer of 1 or 2, and q may be an integer ranging from 2 to 4.

According to one preferred embodiment of the present invention, r may be an integer ranging from 1 to 3, preferably an integer of 1 or 2.

According to one preferred embodiment of the present invention, m and n may be each independently an integer of 1 or 2. Preferably, both m and n may be an integer of 1.

According to one preferred embodiment of the present invention, R₁ may be a carbamoyl group (-C( = O)(NH₂)).

According to one preferred embodiment of the present invention, R₄ may be an amine group (-NH2), a carbamoyl group, or a hydroxyl group (-OH).

The compound of the present invention may include one or more selected from the group consisting of the following compounds, but the present invention is not limited thereto:

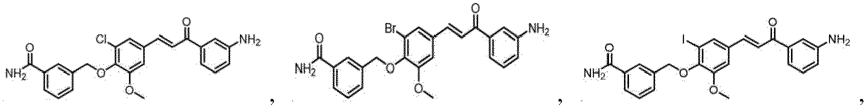

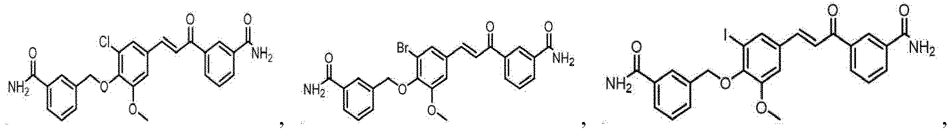

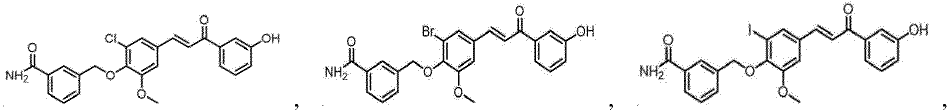

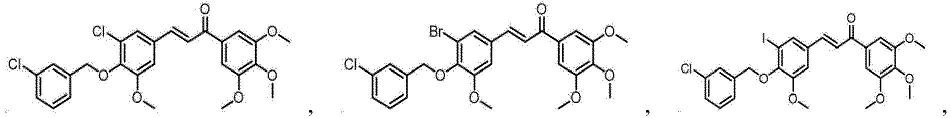

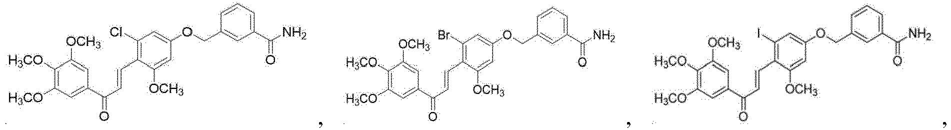

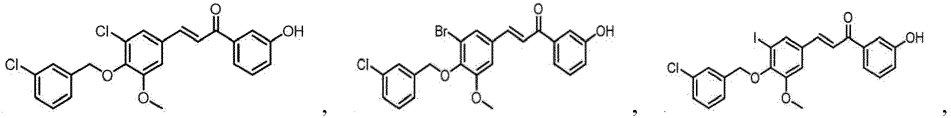

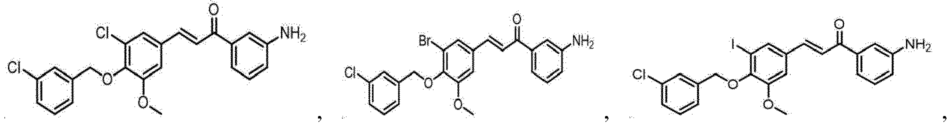

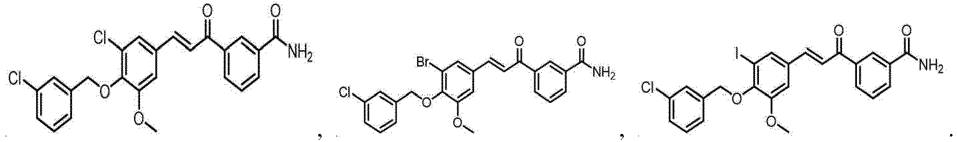

According to one preferred embodiment of the present invention, the compound may be one of the following compounds, but the present invention is not limited thereto:

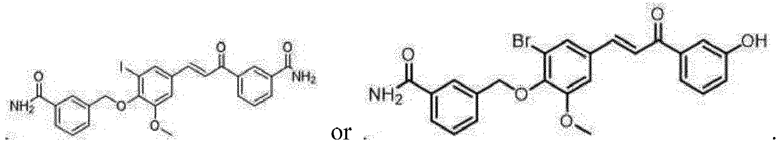

The compounds of the present invention have superior effects in inhibiting a histone acetyltransferase, compared to the existing histone acetyltransferase inhibitors, because the effects of the compounds are improved so that additional hydrogen bonds can be formed with R1410, T1411 and W1466; or R1410 and T1411 of the histone acetyltransferase p300.

According to another embodiment of the present invention, there is provided a compound for inhibiting histone acetyltransferase p300, which is represented by Formula 1.

In the compound for inhibiting HAT p300 according to the present invention, the compound, the histone acetyltransferase p300, and the like are as previously described for the compounds, and thus a description thereof will be omitted in order to avoid undue complexity of the specification.

According to still another embodiment of the present invention, there is provided a composition for preventing, ameliorating or treating a histone acetyltransferase p300-associated disease.

The composition of the present invention may be used as a pharmaceutical composition; a food composition; or a cosmetic composition.

The composition of the present invention includes, as an active ingredient, a compound selected from a novel compound represented by Formula 1 of the present invention, and a pharmaceutically acceptable salt, an optical isomer, a hydrate, and a solvate thereof. Therefore, because the composition of the present invention includes the novel compound, which effectively inhibits the HAT p300, as the active ingredient, the composition may have an effect of preventing, ameliorating or treating a patient with a histone acetyltransferase p300-associated disease in which the expression of HAT p300 increases, for example, fibrosis.

In the composition of the present invention, the compound corresponding to the active ingredient is as previously described for the compounds, and thus a description thereof will be omitted in order to avoid undue complexity of the specification.

The histone acetyltransferase p300-associated disease of the present invention may include all types of diseases that develop when an expression level of the HAT p300 excessively increases or HAT p300 activity excessively increases, compared to the normal control in which the disease does not develop.

In the present invention, the term "fibrosis" refers to a disease in which abnormal production, accumulation and deposition of an extracellular matrix by fibroblasts occur, and may include fibrosis in all types of organs as long as the collagen matrix may be abnormally accumulated by injury or inflammation which may cause the structural and functional change in various tissues. Preferably, the fibrosis may be fibrosis in at least one organ selected from the group consisting of the kidneys, liver, lungs, heart, bone or bone marrow, and skin, but the present invention is not limited thereto. For the purpose of the present invention, the fibrosis may be induced by a phenomenon in which the expression of a gene associated with the fiberization caused by transforming growth factor-β (TGF-β) whose expression level increases by means of the acetyltransferase p300, such as for example a collagen gene, is promoted, or may be induced by the absence of an enzyme capable of recovering cells from the injury by which the fiberization may be induced, but the present invention is not limited thereto.

The fibrosis of the present invention may include one or more selected from the group consisting of pulmonary fibrosis, uterine myoma, myelofibrosis, liver fibrosis, heart fibrosis, multiple sclerosis, kidney fibrosis, cystic fibrosis, neutropenia, skeletal muscle fibrosis, scleroderma, dermatomyositis, mediastinal fibrosis, and splenic fibrosis caused by sickle-cell anemia, and may preferably be pulmonary fibrosis, but the present invention is not limited thereto.

In the present invention, the term "pulmonary fibrosis" refers to a process of (fibrous) tissue development in which a scar is produced due to the excessive formation and development (fibrosis) of fibrous connective tissue in the lung. Specifically, the pulmonary fibrosis is a chronic disease that causes swelling and scars in the interstitial tissue of the alveolus and lung. Inflammation may be caused when healthy tissue is replaced with such a scar tissue. Therefore, chronic inflammation may be considered to be the precursor of fibrosis. Such damage to lung tissue may make the lung become stiff, and make it difficult for a subject to breathe by him/herself.

In the present invention, the pulmonary fibrosis may include idiopathic pulmonary fibrosis, nonspecific interstitial pneumonia, acute interstitial pneumonia, cryptogenic organizing pneumonia, a respiratory bronchiolitis-associated interstitial lung disease, desquamative interstitial pneumonia, lymphoid interstitial pneumonia, interstitial pulmonary fibrosis, and diffuse pulmonary fibrosis. Preferably, the pulmonary fibrosis may be idiopathic pulmonary fibrosis, but the present invention is not limited thereto.

The pulmonary fibrosis of the present invention may be induced by various causes, for example, microscopic damage in the lung induced by the inhalation of fine particles (asbestos, rock dust, metal dust, particles present in cigarette smoke, silica dust, and the like). Also, the pulmonary fibrosis may develop by secondary effects of other diseases (autoimmune disorders, viral or bacterial infections, and the like), and may also be caused by certain drugs such as cytotoxic agents (bleomycin, busulfan, methotrexate, and the like); antibiotics (nitrofurantoin, sulfasalazine, and the like); antiarrhythmic agents (amiodarone, tocainide, and the like); antiinflammatory drugs (gold, penicillamine, and the like); and controlled substances (narcotics, cocaine, heroin, and the like). Also, the idiopathic pulmonary fibrosis may be induced by other unknown causes in addition to the above-described causes.

In the present invention, the term "prevention" may encompass, without any limitation, all types of actions of blocking symptoms caused by a histone acetyltransferase p300-associated disease, for example fibrosis, or suppressing or delaying the symptoms thereof, using the composition of the present invention.

In the present invention, the term "treatment" may encompass, without any limitation, all types of actions performed to improve or ameliorate the symptoms caused by a histone acetyltransferase p300-associated disease, for example fibrosis, using the composition of the present invention.

In the present invention, the term "improvement" may encompass, without any limitation, all types of actions of improving or ameliorating the symptoms caused by a histone acetyltransferase p300-associated disease, for example fibrosis, using the composition of the present invention.

The pharmaceutical composition of the present invention may be formulated and used in the form of an oral formulation (such as a powder, a granule, a capsule, a pill, an aqueous suspension, and the like), a preparation for external use, a suppository, and a sterile injectable solution according to conventional methods, but the present invention is not limited thereto. Preferably, the pharmaceutical composition may be formulated for administration or inhalation administration into the organ; or for use as an injection, but the present invention is not limited thereto. For the purpose of the present invention, the pharmaceutical composition is preferably formulated for inhalation administration so that the active ingredient can reach a target organ with suitable yield for prevention or treatment when the fibrosis develops in the respiratory system such as the lungs.

The pharmaceutical composition of the present invention may include a pharmaceutically acceptable carrier. Upon oral administration, a binder, a lubricant, a disintegrating agent, an excipient, a solubilizing agent, a dispersing agent, a stabilizing agent, a suspending agent, a pigment, a flavoring agent, and the like may be used as the pharmaceutically acceptable carrier. In the case of injection, it may be mixed with a buffer, a preservative, a pain relieving agent, a solubilizing agent, an isotonic agent, a stabilizing agent, and the like, and used. For topical administration, a base, an excipient, a lubricant, a preservative, and the like may be used. The formulation of the pharmaceutical composition according to the present invention may be mixed with the pharmaceutically acceptable carrier as described above, and then prepared into various forms. Upon oral administration, for example, the formulation may be prepared into forms such as a pill, a troch, a capsule, an elixir, a suspension, a syrup, a wafer, and the like. For injection, the formulation may be prepared into unit dosage ampoules or multi-dosage forms. Also, the formulation may be prepared into other solutions, suspensions, pills, capsules, sustained-release preparations, and the like.

Meanwhile, examples of the carrier, excipient, and diluent suitable for preparations that may be used herein include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, or the like. Also, the pharmaceutical composition may further include a filler, an anti-agglomerating agent, a lubricant, a wetting agent, a flavoring agent, an emulsifying agent, a preservative, and the like.

A route of administration of the pharmaceutical composition according to present invention may include, but is not limited to, oral, intravenous, intramuscular, intraarterial, intramedullary, intrathecal, intracardiac, transdermal, subcutaneous, intraperitoneal, intranasal, enteral, local, sublingual, or rectal administration. Oral or parenteral administration is preferred.

In the present invention, the term "parenteral" encompasses subcutaneous, intradermal, intravenous, intramuscular, intraarticular, intrasynovial, intrasternal, intrathecal, intralesional and intracranial injections or infusions. Also, the pharmaceutical composition of the present invention may be administered in the form of a suppository for rectal administration.

The pharmaceutical composition of the present invention may vary according to various factors including the activity of the specific compound used, the age, body weight, general health, and sex of a patient, diet, an administration time, a route of administration, an excretion rate, a drug formulation, and the severity of a certain disease to be prevented or treated, and a dose of the pharmaceutical composition may vary depending on the condition and body weight of a patient, the severity of a disease, the type of a drug, a route of administration, and an administration duration, but may be suitably selected by those of ordinary skill in the art, and may be administered at 0.0001 to 50 mg/kg or 0.001 to 50 mg/kg per day. The pharmaceutical composition of the present invention may be administered once a day or several times in divided doses. The dose is not intended to limit the scope of the present invention in any way. The pharmaceutical composition according to the present invention may be formulated into a pill, a sugar-coated tablet, a capsule, a liquid, a gel, a syrup, a slurry, or a suspension.

When the food composition of the present invention is prepared in the form of beverages, the food composition is used without particular limitation as long as the food composition is included at a given ratio. Like conventional beverages, the food composition may contain various flavoring agents, natural carbohydrates, or the like as additional components. Specifically, the food composition may include monosaccharides such as glucose, and the like; disaccharides such as fructose, and the like; polysaccharides such as sucrose, and the like; conventional sugars such as dextrin, cyclodextrin, and the like; and sugar alcohols such as xylitol, sorbitol, erythritol, and the like, as the natural carbohydrates. The food composition may include natural flavoring agents (thaumatin, stevia extracts (for example, rebaudioside A, glycyrrhizin, and the like), synthetic flavoring agents (saccharin, aspartame, and the like), and the like as the flavoring agent.

The food composition of the present invention may further include various nutrients, vitamins, minerals (electrolytes), a flavoring agent (including synthetic flavoring agents and natural flavoring agents), a coloring agent, pectic acid and a salt thereof, alginic acid and a salt thereof, an organic acid, a prospective colloid thickening agent, a pH regulator, a stabilizing agent, a preservative, glycerin, an alcohol, a carbonating agent used for carbonated beverages, and the like.

The components included in the food composition of the present invention may be used alone or in combination. The proportions of the additives are not the key factor of the present invention, but may be selected within a range of 0.1 to approximately 50 parts by weight, based on 100 parts by weight of the food composition of the present invention, but the present invention is not limited thereto.

The cosmetic composition of the present invention may be prepared into forms of a face lotion, a nourishing lotion, a nourishing essence, a massage cream, a beauty bath additive, a body lotion, a body milk, a bath oil, a baby oil, a baby powder, a shower gel, a shower cream, a sun screen lotion, a sun screen cream, a suntan cream, a skin lotion, a skin cream, sunscreen cosmetics, a cleansing milk, depilatories, a face and body lotion, a face and body cream, a skin whitening cream, a hand lotion, a hair lotion, a cosmetic cream, a jasmine oil, a bath soap, a liquid soap, a beauty soap, a shampoo, a hand sanitizer (i.e., a hand cleaner), a non-medicated soap, a cream soap, a facial wash, a cleaner for whole body, a scalp cleanser, a hair rinse, a toilet soap, a tooth whitening gel, a toothpaste, and the like. The composition of the present invention may further include a solvent commonly used to prepare the cosmetic composition, or a suitable carrier, excipient, or diluent.

For example, types of the solvent that may be further included in the cosmetic composition of the present invention, which may be used herein, may include water, saline, DMSO or a combination thereof. Also, purified water, an oil, a wax, a fatty acid, a fatty acid alcohol, a fatty acid ester, a surfactant, a humectant, a thickening agent, an antioxidant, a viscosity stabilizing agent, a chelating agent, a buffer, a lower alcohol, and the like may be included as the carrier, excipient, or diluent, but the present invention is not limited thereto. Also, the cosmetic composition may include a whitening agent, a moisturizing agent, vitamins, a sunscreen, a perfume, a dye, an antibiotic, an antibacterial agent, an antifungal agent, and the like, when necessary.

Hydrogenated vegetable oil, castor oil, cotton seed oil, olive oil, palm-kernel oil, jojoba oil, avocado oil, and the like may be used as the oil of the present invention, and beeswax, spermaceti wax, carnauba, candelilla, montan, ceresin, liquid paraffin, lanolin, and the like may be used as the wax.

Stearic acid, linoleic acid, linolenic acid, oleic acid, and the like may be used as the fatty acid of the present invention, cetyl alcohol, octyl dodecanol, oleyl alcohol, panthenol, lanolin alcohol, stearyl alcohol, hexadecanol, and the like may be used as the fatty acid alcohol, and isopropyl myristate, isopropyl palmitate, butyl stearate, and the like may be used as the fatty acid ester. A cationic surfactant, an anionic surfactant, and a non-ionic surfactant known in the art may be used as the surfactant. In this case, surfactants derived from natural products are preferred, if possible. In addition, the cosmetic composition may include a humectant, a thickening agent, an antioxidant, and the like, which are widely known in the field of cosmetics. In this case, the types and amounts of the humectant, the thickening agent, and the antioxidant are as known in the related art.

According to yet another embodiment of the present invention, there is provided a method of preventing, ameliorating or treating a histone acetyltransferase p300-associated disease, which includes administering the composition of the present invention to a target subject.

In the present invention, the general contents of the histone acetyltransferase p300-associated disease, the prevention, the improvement, the treatment, the compound, and the composition are as described above, and thus a description thereof will be omitted in order to avoid undue complexity of the specification.

In the present invention, the term "subject" is a subject suspected of developing the histone acetyltransferase p300-associated disease. In this case, the subject suspected of developing the histone acetyltransferase p300-associated disease refers to a mammal (including a human who has developed or may develop the corresponding disease), such as a rat, livestock, and the like. However, the subject includes, but is not limited to, subjects who may be treated using a fusion protein of the present invention or the composition including the same.

The method of the present invention may include administering a pharmaceutically effective amount of the active ingredient. A suitable total daily dosage may be determined by a physician within the range of sound medical judgment, and administered once or divided into several doses. However, for the purpose of the present invention, a specific therapeutically effective amount for a specific patient is preferably applied depending on various factors including the type and degree of response to be accomplished, a specific composition (including whether it may be optionally used together with another preparation), the age, body weight, general health condition, gender, and diet of a patient, an administration time, a route of administration, a secretion rate of the composition, a treatment duration, and a drug used with or concurrently with the specific composition, and similar factors well known in the field of medicine.

It should be understood that the "combination" used herein refers to concurrent, separate or sequential administration. In the case of sequential or separate administration, the intervals of the administration of a secondary component should be such that the beneficial effect of the combination therapy is not lost.

In the present invention, the dose of the fusion protein of the present invention administered may be in a range of approximately 0.0001 µg to 500 mg per kg of a patient's body weight, but the present invention is not limited thereto.

### [Advantageous Effects]

The present invention relates to a novel compound which enables additional hydrogen bonding with a specific amino acid position of histone acetyltransferase (HAT) p300 through the structural analysis of the HAT p300. The novel compound of the present invention has an excellent inhibitory effect on HAT p300 activity, and thus can be very effectively used to prevent, ameliorate or treat a disease associated with the activation of HAT p300, such as fibrosis.

### [Description of Drawings]

FIGS. 1 to 3 show the results of confirming, through immunohistochemical staining, expression levels of histone acetyltransferase (HAT) proteins (i.e., p300 (histone acetyltransferase p300; FIG. 1), GCN5 (histone acetyltransferase GCN5; FIG. 2), and PCAF (P300/CBP-associated factor; FIG. 3)) in the tissue from a patient with idiopathic pulmonary fibrosis according to one embodiment of the present invention.
FIG. 4 shows the results of confirming the degree of inhibition of HAT activity of Candidates 1 to 67, HAT-24, HAT-26, and HAT-28 that are PCAF inhibitors according to one embodiment of the present invention.
FIG. 5 shows the results of confirming the degree of inhibition of HAT activity of Candidates 1 to 14 according to one embodiment of the present invention.
FIG. 6 shows a Lys-CoA molecule model of a HAT p300 domain and a substrate inhibitor using a molecular docking simulation according to one embodiment of the present invention.
FIG. 7 shows the results of analyzing main residues that participate in an intermolecular bond between the HAT p300 domain and the substrate inhibitor using the molecular docking simulation according to one embodiment of the present invention.
FIG. 8 shows the results of analyzing main residues that participate in a bond between Candidate 12 and the HAT p300 domain using the molecular docking simulation according to one embodiment of the present invention.
FIG. 9 shows the results of confirming the degree of inhibition of histone acetyltransferase activity of Synthesis Examples 1 to 19 (A 1 to A 19) according to one embodiment of the present invention.
FIG. 10 shows the results of confirming the degree of inhibition of histone acetyltransferase activity of Synthesis Example 6 and 9 (A25 and A27) according to one embodiment of the present invention.
FIGS. 11A to 11C show the results of analyzing main residues that participate in a bond between Synthesis Examples 6 and 8 (A25 and A27) and the HAT p300 domain using the molecular docking simulation according to one embodiment of the present invention.

### [Best Mode]

According to one embodiment of the present invention, there is provided a compound selected from a compound represented by the following Formula 1, and a pharmaceutically acceptable salt, an optical isomer, a hydrate, and a solvate thereof: 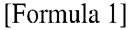 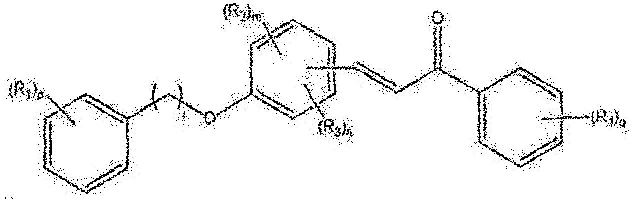 wherein p and q are each independently an integer ranging from 1 to 5; r is an integer ranging from 1 to 4; m and n are each independently an integer ranging from 1 to 3, provided that m + n is not greater than 4; R₁ is a carbamoyl group (-C( = O)(NH₂)) or a halogen, wherein when R₁ is present in a plural number, they are the same or different from each other; R2 and R3 are each independently a C₁-C₆ alkoxy group or a halogen, wherein when each of R2 and R3 is present in a plural number, they are the same or different from each other; and R4 includes any one selected from the group consisting of a C₁-C₆ alkoxy group, an amine group (-NH2), a carbamoyl group, and a hydroxyl group (-OH), wherein when R4 is present in a plural number, they are the same or different from each other.

According to another embodiment of the present invention, there is provided a composition for preventing, ameliorating or treating a HAT p300-associated disease, which includes, as an active ingredient, a compound selected from a compound represented by Formula 1, and a pharmaceutically acceptable salt, an optical isomer, a hydrate, and a solvate thereof.

According to still another embodiment of the present invention, there is provided a method of preventing, ameliorating or treating a HAT p300-associated disease, which includes, as an active ingredient, a compound selected from a compound represented by Formula 1, and a pharmaceutically acceptable salt, an optical isomer, a hydrate, and a solvate thereof.

### [Mode for Invention]

Hereinafter, the present invention will be described in detail with reference to the following examples. It will be apparent to those skilled in the art that the following examples are merely provided to exemplify the present invention, and are not intended to limit the scope of the present invention without departing from the scope of the present invention.

### EXAMPLES

### [Example 1] Confirmation of protein expression level in tissue of fibrosis patient

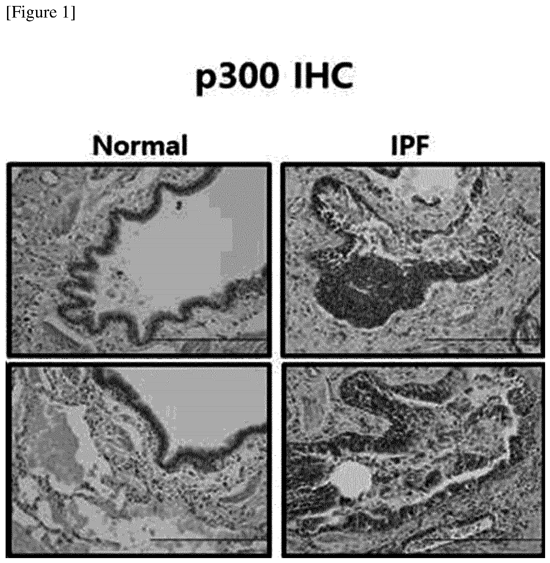

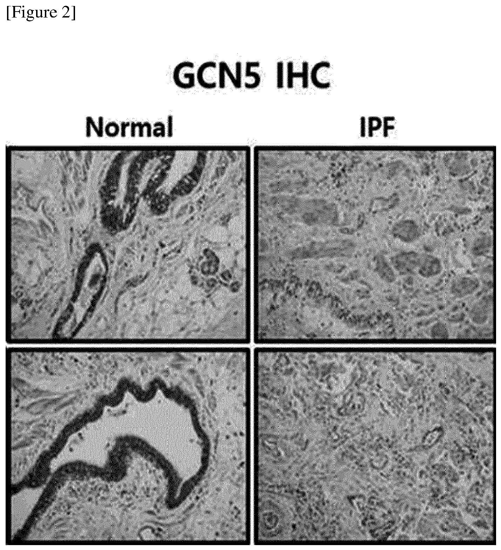

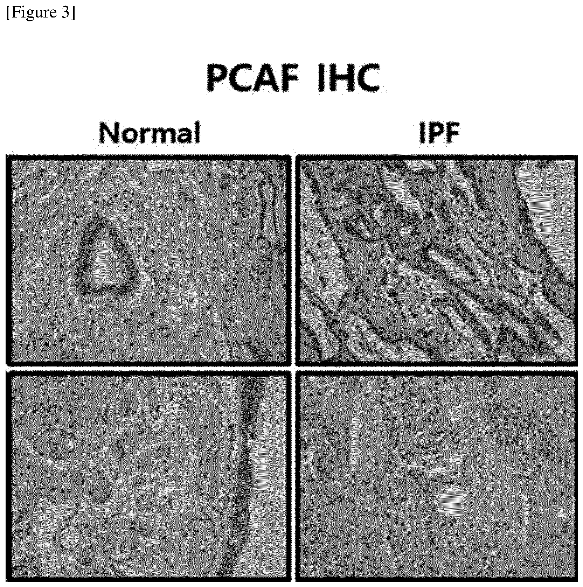

Tissues obtained from a patient with idiopathic pulmonary fibrosis or a normal person were fixed in 10% formalin, and embedded in paraffin, and a 7 µm-thick section was attached to a slide. Thereafter, the section was deparaffinized using xylene, and treated with a high concentration to a low concentration of ethanol. Then, immunostaining was performed using antibodies specific to HAT p300 (histone acetyltransferase p300), GCN5 (histone acetyltransferase GCN5), and PCAF (P300/CBP-associated factor), and an expression level of each proteins was measured using an optical microscope. The results are shown in FIGS. 1 to 3.

As shown in FIGS. 1 to 3, it was confirmed that among the histone acetyltransferases (hereinafter referred to as ‴HAT") p300, GCN5, and PCAF, the expression of p300 increased in the tissue from the patient with idiopathic pulmonary fibrosis, compared to the tissue obtained from the normal person.

Based on the results, it can be seen that the fibrosis is able to be effectively treated when the expression or function of p300 is specifically inhibited because especially p300 among the histone acetyltransferases is present at a high level in the fibrosis such as idiopathic pulmonary fibrosis, compared to the normal tissue.

### [Example 2] Screening of p300 activity inhibitors

### [2-1] Primary screening

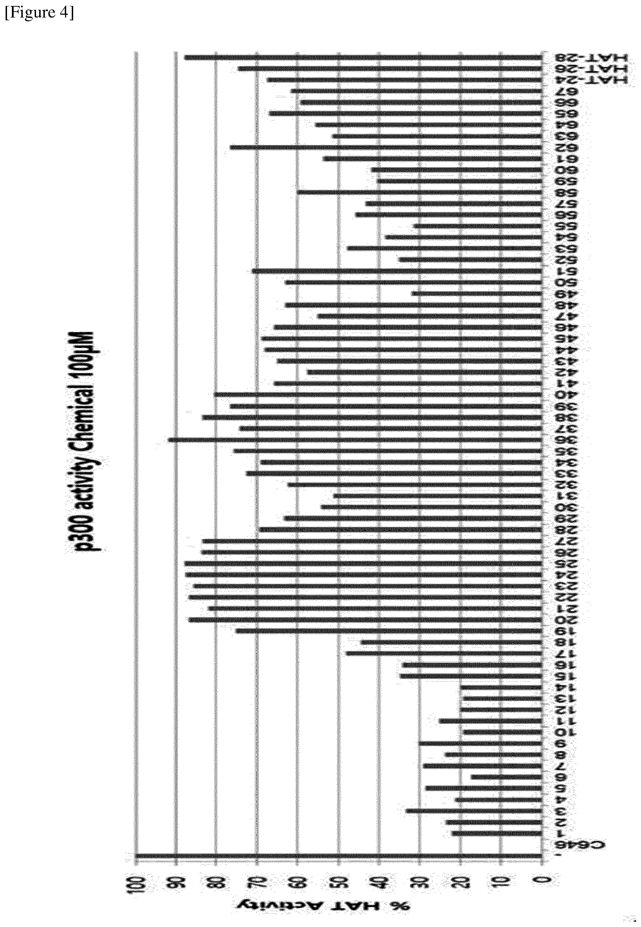

Candidates 1 to 67, which are HAT inhibitors manufactured based on the PCAF structure, and HAT-24, HAT-26, and HAT-28 were diluted to 100 µM, and the degree of inhibition of HAT activity of p300 (inhibitory activity) was then determined using a kit for measuring HAT activity (Biovision, Cat No. K332, U.S.A) according to the method provided by the manufacturer. The results are shown in FIG. 4. Here, the tissue was treated with C646 (HAT inhibitor) as a positive control.

As shown in FIG. 4, 80% of the HAT activity was inhibited by Candidates 1 to 14, but Candidate 15 to 67, HAT-25, HAT-26, and HAT-28 had a HAT activity inhibitory effect of only 20% to 50%.

### [2-2] Secondary screening

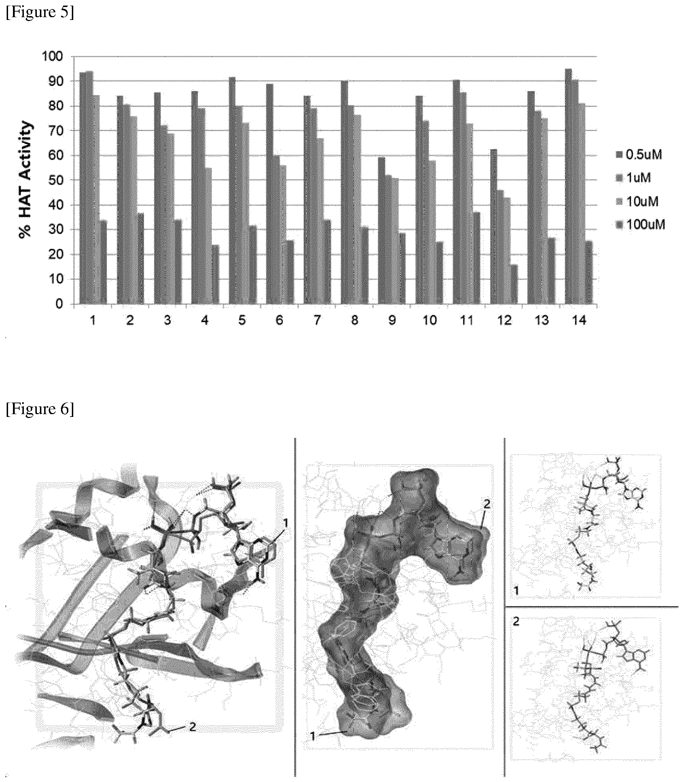

Candidates 1 to 14 having a good HAT activity inhibitory effect in Section [2-1] were diluted to concentrations of 0.5 µM, 1 µM, 10 µM, and 100 µM. Thereafter, the degree of inhibition of HAT activity was determined in the same manner as in Section [2-1]. The results are shown in FIG. 5 and Table 1.

**[Table 1]**

| | | | | | | | | | | | | | | |
|---|---|---|---|---|---|---|---|---|---|---|---|---|---|---|
| Candida te | 1 | 2 | 3 | 4 | 5 | 6 | 7 | 8 | 9 | 10 | 11 | 12 | 13 | 14 |
| IC₅₀ (µM) | 50.84 | 44.09 | 28.9 | 12.12 | 32.4 | 10.14 | 27.65 | 35.52 | 9.01 | 13.12 | 41.67 | 0.953 | 32.84 | 35.82 |

As shown in FIG. 5 and Table 1, it was confirmed that among Candidates 1 to 14, Candidate 12 (HAT-12) had an ICso of 0.953.

### [Example 3] Structural analysis of Candidate 12

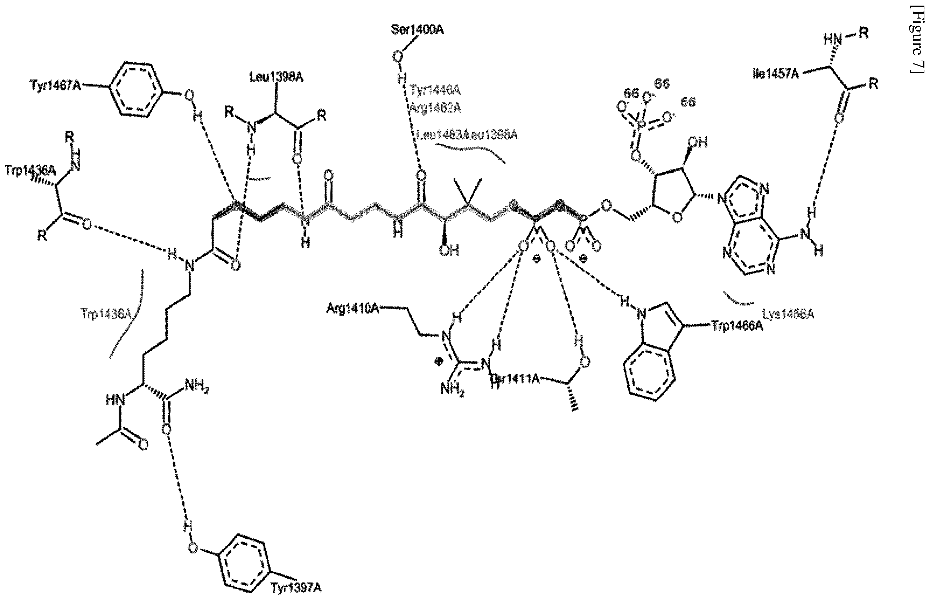

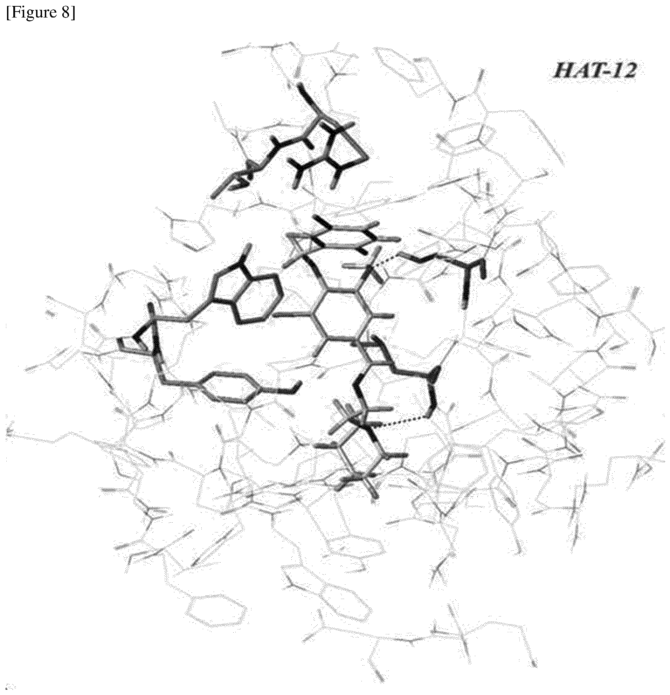

To obtain information on the structure-activity correlation of a candidate through a molecular docking simulation, a molecule model was established using a co-crystal structure (pdb: 3biy) of a HAT p300 domain and its substrate inhibitor Lys-CoA. The Lys-CoA was finally re-docked into the molecule model thus established, and the co-crystal structures were compared. As a result, it was confirmed that the candidates had a similar binding pattern. From the results, the accuracy of the docking results was evaluated (FIG. 6), the main residues participating in the binding between the HAT p300 domain and the Lys-CoA were analyzed (FIG. 7), and a molecular docking simulation was performed with Candidate 12 (HAT-12) (FIG. 8).

As shown in FIG. 6, it was confirmed that there was a high similarity between a predicted binding pattern of the HAT p300 domain-ligand (Lys-CoA) and the co-crystal structure.

As shown in FIG. 7, it was confirmed that when the main residues participating in the binding between the Lys-CoA and the HAT p300 domain were analyzed, hydrogen bonds were observed at R1410, T1411, W1466, Y1467, L1398, S1400, 11457, W1436, and Y1397. Among these, the interactions with R1410, T1411, W1466, and Y1467 were important for drug inhibitory activity. Especially, it can be seen that W1466 played a key role in drug inhibitory activity (see, for example, Erin M.Bowers et al., Virtual Ligand Screening of the p300/CBP Histone Acetyltransferase: Identification of a Selective Small Molecule Inhibitor, Chem Biol. 2010 May 28; 17(5): 471-82).

As shown in FIG. 8, it was confirmed that when the main residues participating in the binding between Candidate 12 and the HAT p300 domain were analyzed, the interactions with R1410, T1411, W1466, and Y1467 were not observed, but e hydrogen bonds were formed with L1398 and S1400.

From the results, it can be expected that L1398 and S1400 also play an important role in drug inhibitory activity. Also, it can be seen that when the additional hydrogen bonding with R1410, T1411, W1466, and Y1467 was hindered, HAT p300 inhibitory activity was further enhanced.

Based on these results, Synthesis Examples 1 to 24 (A20 to A43) having the above-described characteristics were prepared to further enhance HAT p300 inhibitory activity, as follows.

### [Synthesis Examples 1 to 24] Preparation of novel compounds for inhibiting histone acetyltransferase p300

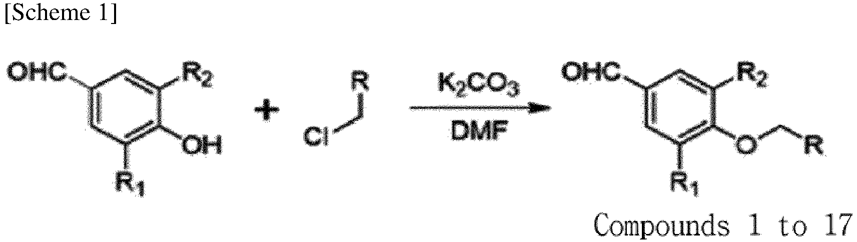

### [Synthesis Method 11 Compounds 1 to 17

Substituted 4-hydroxybenzaldehyde (1 equivalent) and its corresponding substituted benzyl chloride (1 equivalent), and K₂CO₃ (1 equivalent) were cultured in a DMF solvent at 80 °C for an hour. The reaction mixture was cooled to room temperature, and extracted using ethyl acetate. The extract was washed with water, NaHCO₃, and brine, and then dried over MgSO₄. Thereafter, the solvent was completely removed under reduced pressure, and silica gel chromatography was then performed to obtain Compounds 1 to 17 as O-benzylated compounds (see Table 2).

**[Table 2]**

| Compound | R | R₁ | R₂ |
|---|---|---|---|
| 1 | 3-chlorophenyl | OCH₃ | Cl |
| 2 | 3- chlorophenyl | OCH₃ | Br |
| 3 | 3- chlorophenyl | OCH₃ | I |
| 4 | 3-ethoxycarbonylphenyl | OCH₃ | Cl |
| 5 | 3-ethoxycarbonylphenyl | OCH₃ | Br |
| 6 | 3-ethoxycarbonylphenyl | OCH₃ | I |
| 7 | 3-cyanophenyl | OCH₃ | Cl |
| 8 | 3-cyanophenyl | OCH₃ | Br |
| 9 | 3-cyanophenyl | OCH₃ | I |
| 10 | 3-fluorophenyl | OCH₃ | Cl |
| 11 | 3- chlorophenyl | OCH₃ | H |
| 12 | 4-carbamoylphenyl | OCH₃ | Cl |
| 13 | 4- carbamoylphenyl | OCH₃ | Br |
| 14 | 4- carbamoylphenyl | OCH₃ | I |
| 15 | 3- carbamoylphenyl | OCH₃ | Cl |
| 16 | 3- carbamoylphenyl | OCH₃ | Br |
| 17 | 3- carbamoylphenyl | OCH₃ | I |

### [Compound 1] 3-chloro-4-(3-chlorobenzyloxy)-5-methoxybenzaldehyde

5-chlorovanillin (1.00 g, 5.36 mmol) and 3-chlorobenzyl chloride (0.70 g, 5.36 mmol) were used to obtain Compound 1 as an ivory solid (1.50 g, 96.5%).

R_{f} 0.54 (ethyl acetate:n-hexane = 1:3); ¹H-NMR (CDCl₃, 400 MHz) δ 3.95 (s, 3H), 5.13 (s, 2H), 7.30-7.32 (m, 2H), 7.36 (d, J = 2.0 Hz, 1H), 7.37-7.39 (m, 1H), 7.51 (d, J = 2.0 Hz, 1H), 7.53-7.54 (m, 1H), 9.86 (s, 1H); ¹³C-NMR (CDCl₃, 100 MHz) 56.5, 74.3, 109.6, 125.9, 126.4, 128.5, 128.6, 129.4, 129.9, 132.9, 134.5, 138.7, 149.4, 154.5, 190.1 ppm.

### [Compound 2] 3-bromo-4-(3-chlorobenzyloxy)-5-methoxybenzaldehyde

5-bromovanillin (1.00 g, 4.33 mmol) and 3-chlorobenzyl chloride (0.58 g, 5.36 mmol) were used to obtain Compound 2 as an ivory solid (1.50 g, 96.5%).

R_{f} 0.65 (ethyl acetate:n-hexane = 1:3); ¹H-NMR (CDCl₃, 400 MHz) δ 3.94 (s, 3H), 5.12 (s, 2H), 7.30-7.32 (m, 2H), 7.38 (dd, J = 8.4, 1.2 Hz, 1H), 7.40 (d, J = 2.0 Hz, 1H), 7.54-7.56 (m, 1H), 7.66 (d, J = 2.0 Hz, 1H), 9.85 (s, 1H); ¹³C-NMR (CDCl₃, 100 MHz) 56.5, 74.2, 110.3, 118.5, 126.5, 128.6, 129.0, 129.9, 133.5, 134.5, 138.7, 150.4, 154.4, 190.0 ppm.

### [Compound 3] 4-((3-chlorobenzyl)oxy)-3-iodo-5-methoxybenzaldehyde

5-iodovanillin (1.00 g, 3.60 mmol) and 3-chlorobenzyl chloride (0.58 g, 5.36mmol) were used to obtain Compound 3 as an ivory solid (1.423 g, 98.1%).

R_{f} 0.67 (ethyl acetate:n-hexane = 1:3); ¹H-NMR (CDCl₃, 400 MHz) δ 3.94 (s, 3H), 5.10 (s, 2H), 7.31-7.33 (m, 2H), 7.41 (dd, J = 8.4, 1.2 Hz, 1H),.7.43 (d, J = 1.6 Hz, 1H), 7.57-7.58 (m, 1H), 7.87 (d, J = 1.6 Hz, 1H), 9.84 (s, 1H); ¹³C-NMR (CDCl₃, 100 MHz) 56.4, 74.0, 92.8, 111.2, 126.6, 128.7, 128.8, 129.9, 134.4, 134.5, 135.1, 138.7, 152.9, 153.2, 189.9 ppm.

### [Compound 4] Ethyl 3-((2-chloro-4-formyl-6-methoxyphenoxy)methyl)benzoate

5-chlorovanillin (1.00 g, 5.36 mmol) and ethyl 3-(chloromethyl)benzoate (1.06 g, 5.36 mmol) were used to obtain Compound 4 as a yellow solid (1.18 g, 63.1%).

R_{f} 0.39 (ethyl acetate:n-hexane = 1:3); ¹H-NMR (CDCl₃, 400 MHz) δ 1.41 (t, J = 7.2 Hz, 3H), 3.95 (s, 3H), 4.39 (q, J = 7.2 Hz, 2H), 5.21 (s, 2H), 7.30 (ddd, J = 7.6, 1.2, 1.2 Hz, 1H), 7.36 (d, J = 1.6 Hz, 1H), 7.46 (dd, J = 7.6, 7.6 Hz, 1H), 7.50 (d, J = 2.0 Hz, 1H), 8.02 (ddd, J = 7.6, 1.2, 1.2 Hz, 1H), 8.18 (dd, J = 1.2, 1.2 Hz, 1H), 9.85 (s, 1H); ¹³C-NMR (CDCl₃, 100 MHz) 14.6, 56.5, 61.3, 74.7, 109.6, 126.0, 128.7, 129.4, 129.6, 129.7, 130.9, 132.8, 132.9, 137.1, 149.5, 154.6, 166.6, 190.2 ppm.

### [Compound 5] Ethyl 3-((2-bromo-4-formyl-6-methoxyphenoxy)methyl)benzoate

5-bromovanillin (1.00 g, 4.33 mmol) and ethyl 3-(chloromethyl)benzoate (0.86 g, 4.33 mmol) were used to obtain Compound 5 as a pale yellow solid (1.53 g, 90.1%).

R_{f} 0.35 (ethyl acetate:n-hexane = 1:3); ¹H-NMR (CDCl₃, 400 MHz) δ 1.41 (t, *J* = 7.2 Hz, 3H), 3.95 (s, 3H), 4.39 (q, *J* = 7.2 Hz, 2H), 5.21 (s, 2H), 7.40 (d, *J* = 2.0 Hz, 1H), 7.46 (dd, *J* = 7.6, 7.6 Hz, 1H), 7.66 (d, *J* = 2.0 Hz, 1H), 7.75 (ddd, *J* = 7.6, 1.2, 1.2 Hz, 1H), 8.02 (ddd, *J* = 7.6, 1.2, 1.2 Hz, 1H), 8.19 (dd, *J* = 1.2, 1.2 Hz, 1H), 9.85 (s, 1H); ¹³C-NMR (CDCl₃, 100 MHz) 14.6, 56.5, 61.3, 74.6, 110.3, 118.6, 128.7, 129.0, 129.7, 130.9, 133.0, 133.4, 137.1, 150.6, 154.4, 166.6, 190.0 ppm.

### [Compound 6] Ethyl 3-((4-formyl-2-iodo-6-methoxyphenoxy)methyl)benzoate

5-iodovanillin (1.00 g, 3.60 mmol) and ethyl 3-(chloromethyl)benzoate (0.71 g, 3.60 mmol) were used to obtain Compound 6 as a pale yellow solid (1.41 g, 88.8%).

R_{f} 0.39 (ethyl acetate:n-hexane = 1:3); ¹H-NMR (CDCl₃, 400 MHz) δ 1.41 (t, *J* = 7.2 Hz, 3H), 3.95 (s, 3H), 4.39 (q, *J* = 7.2 Hz, 2H), 5.21 (s, 2H), 7.43 (d, *J* = 1.6 Hz, 1H), 7.47 (dd, *J* = 7.6, 7.6 Hz, 1H), 7.78 (ddd, *J* = 7.6, 1.2, 1.2 Hz, 1H), 7.86 (d, *J* = 1.6 Hz, 1H), 8.02 (ddd, *J* = 7.6, 1.2, 1.2 Hz, 1H), 8.22 (dd, *J* = 1.6, 1.6 Hz, 1H), 9.83 (s, 1H); ¹³C-NMR (CDCl₃, 100 MHz) 14.6, 56.3, 61.3, 74.4, 92.8, 111.2, 128.7, 129.7, 129.8, 130.9, 133.1, 134.3, 135.1, 137.1, 152.9, 153.2, 166.6, 189.9 ppm.

### [Compound 7] 3-((2-chloro-4-formyl-6-methoxyphenoxy)methyl)benzonitrile

5-chlorovanillin (1.00 g, 5.36 mmol) and ethyl 3-(bromomethyl)benzonitrile (1.05 g, 5.36 mmol) were used to obtain Compound 7 as a white solid (1.30 g, 80.5%).

R_{f} 0.25 (ethyl acetate:n-hexane = 1:3); ¹H-NMR (CDCl₃, 400 MHz) δ 3.96 (s, 3H), 5.17 (s, 2H), 7.38 (d, *J* = 2.0 Hz, 1H), 7.50 (dd, *J* = 7.6, 7.6 Hz, 1H), 7.52 (d, *J* = 2.0 Hz, 1H), 7.64 (ddd, *J* = 7.6, 1.6, 1.6 Hz, 1H), 7.75 (ddd, *J* = 7.6, 1.6, 1.6 Hz, 1H), 7.84 (dd, *J* = 1.6, 1.2 Hz, 1H), 9.87 (s, 1H); ¹³C-NMR (CDCl₃, 100 MHz) 56.6, 73.8, 109.6, 112.8, 118.9, 126.0, 129.4, 129.5, 131.8, 132.1, 132.5, 133.1, 138.4, 149.1, 154.5, 190.1 ppm.

### [Compound 8] 3-((2-bromo-4-formyl-6-methoxyphenoxy)methyl)benzonitrile

5-bromovanillin (1.00 g, 4.33 mmol) and ethyl 3-(bromomethyl)benzonitrile (0.85 g, 4.33 mmol) were used to obtain Compound 8 as a white solid (1.53 g, 83.8%).

R_{f} 0.24 (ethyl acetate:n-hexane = 1:3); ¹H-NMR (CDCl₃, 400 MHz) δ 3.95 (s, 3H), 5.16 (s, 2H), 7.42 (d, *J* = 2.0 Hz, 1H), 7.50 (dd, *J* = 8.0, 7.6 Hz, 1H), 7.64 (ddd, *J* = 7.6, 2.0 1.6 Hz, 1H), 7.67 (d, *J* = 1.6 Hz, 1H), 7.70 (ddd, *J* = 8.0, 1.6, 1.2 Hz, 1H), 7.86 (dd, *J* = 1.6, 1.6 Hz, 1H), 9.86 (s, 1H); ¹³C-NMR (CDCl₃, 100 MHz) 56.5, 73.6, 110.3, 112.8, 118.5, 118.9, 129.0, 129.5, 131.9, 132.1, 132.6, 133.7, 138.4, 150.1, 154.3, 189.9 ppm.

### [Compound 9] 3-((4-formyl-2-iodo-6-methoxyphenoxy)methyl)benzonitrile

5-iodovanillin (1.00 g, 3.60 mmol) and ethyl 3-(bromomethyl)benzonitrile (0.71 g, 3.60 mmol) were used to obtain Compound 9 as a white solid (1.20 g, 84.4%).

R_{f} 0.27 (ethyl acetate:n-hexane = 1:3); ¹H-NMR (CDCl₃, 400 MHz) δ 3.95 (s, 3H), 5.15 (s, 2H), 7.44 (d, *J* = 2.0 Hz, 1H), 7.51 (dd, *J* = 8.0, 7.6 Hz, 1H), 7.65 (ddd, *J* = 7.6, 1.6, 1.2 Hz, 1H), 7.78 (ddd, *J* = 7.6, 1.6, 1.2 Hz, 1H), 7.88 (d, *J* = 2.0 Hz, 1H), 7.89 (dd, *J* = 1.6, 1.2 Hz, 1H), 9.85 (s, 1H); ¹³C-NMR (CDCl₃, 100 MHz) 56.4, 73.4, 92.7, 111.3, 112.8, 118.9, 129.5, 132.0, 132.1, 132.7, 134.6, 135.0, 138.3, 152.5, 153.2, 189.8 ppm.

### [Compound 10] 3-chloro-4-((3-fluorobenzyl)oxy)-5-methoxybenzaldehyde

5-chlorovanillin (1.00 g, 5.36 mmol) and ethyl 3-fluorobenzyl chloride (0.78 g, 5.36 mmol) were used to obtain Compound 10 as a white solid (1.15 g, 72.8%).

R_{f} 0.58 (ethyl acetate:n-hexane = 1:3); ¹H-NMR (CDCl₃, 400 MHz) δ 3.95 (s, 3H), 5.15 (s, 2H), 7.00-7.05 (m, 1H), 7.24-7.28 (m, 2H), 7.34 (dd, *J* = 8.0, 2.0 Hz, 1H), 7.37 (d, *J* = 2.0 Hz, 1H), 7.51 (d, *J* = 2.0 Hz, 1H), 9.85 (s, 1H); ¹³C-NMR (CDCl₃, 100 MHz) 56.5, 74.4, 109.7, 115.3, 115.5, 123.8, 126.0, 130.1, 132.9, 139.3, 149.5, 154.6, 161.8, 164.3, 190.2 ppm.

### [Compound 11] 4-((3-chlorobenzyl)oxy)-3-methoxybenzaldehyde

Vanillin (1.00 g, 6.57 mmol) and 3-methoxybenzyl chloride (1.03 g, 6.57 mmol) were used to obtain Compound 11 as a yellow semisolid (1.48 g, 99.6%).

R_{f} 0.45 (ethyl acetate:n-hexane = 1:3);¹H-NMR (CDCl₃, 400 MHz) δ 3.95 (s, 3H), 5.19 (s, 2H), 6.95 (d, *J* = 8.4 Hz, 1H), 7.29-7.32 (m, 3H), 7.39 (dd, *J* = 8.0, 1.6 Hz, 1H), 7.43-7.44 (m, 2H), 9.84 (s, 1H); ¹³C-NMR (CDCl₃, 100 MHz) 56.2, 70.2, 109.7, 112.6, 125.4, 126.6, 127.4, 128.6, 130.2, 130.7, 134.8, 138.3, 150.3, 153.4, 191.0 ppm.

### [Compound 12] 4-((2-chloro-4-formyl-6-methoxyphenoxy)methyl)benzamide

5-chlorovanillin (0.18 g, 0.96 mmol) and 4-(chloromethyl)benzamide (0.16 g, 0.96 mmol) were used to obtain Compound 12 as a white solid (0.22 g, 73.2%).

R_{f} 0.25 (ethyl acetate:n-hexane = 1:1); ¹H-NMR (400 MHz, DMSO-d₆) δ 3.88 (s, 3H), 5.12 (s, 2H), 7.29 (d, *J* = 1.6 Hz, 1H), 7.44 (d, *J* = 2.0 Hz, 1H), 7.48 (d, *J* = 8.0 Hz, 2H), 7.83 (d, *J* = 8.0 Hz, 2H), 9.78 (s, 1H); ¹³C-NMR (400 MHz, DMSO-d₆) 55.2, 73.7, 109.5, 124.5, 127.2, 127.4, 128.3, 132.0, 133.2, 139.4, 148.4, 153.7, 168.4, 189.4 ppm.

### [Compound 13] 4-((2-bromo-4-formyl-6-methoxyphenoxy)methyl)benzamide

5-bromovanillin (0.18 g, 0.79 mmol) and 4-(chloromethyl)benzamide (0.13 g, 0.79 mmol) were used to obtain Compound 13 as a white solid (0.20 g, 73.2%).

R_{f} 0.16 (ethyl acetate:n-hexane = 1:1); ¹H-NMR (400 MHz, DMSO-d₆) δ 3.88 (s, 3H), 5.11 (s, 2H), 7.34 (d, J = 2.0 Hz, 1H), 7.50 (d, *J* = 8.4 Hz, 2H), 7.60 (d, *J* = 1.6 Hz, 1H), 7.83 (d, *J* = 8.4 Hz, 2H), 9.78 (s, 1H); ¹³C-NMR (400 MHz, DMSO-d₆) 55.7, 73.6, 100.1, 117.6, 127.2, 127.4, 127.5, 132.6, 133.2, 139.4, 149.5, 153.5, 168.4, 189.3 ppm.

### [Compound 14] 4-((4-formyl-2-iodo-6-methoxyphenoxy)methyl)benzamide

5-iodovanillin (0.26 g, 0.94 mmol) and 4-(chloromethyl)benzamide (0.16 g, 0.94 mmol) were used to obtain Compound 14 as a white solid (0.28 g, 72.4%).

R_{f} 0.10 (ethyl acetate:n-hexane = 1:1); ¹H-NMR (400 MHz, DMSO-d₆) δ 3.87 (s, 3H), 5.10 (s, 2H), 7.36 (d, *J* = 2.0 Hz, 1H), 7.53 (d, *J* = 8.0 Hz, 2H), 7.79 (d, *J* = 1.6 Hz, 1H), 7.83 (d, *J* = 8.4 Hz, 2H), 9.76 (s, 1H); ¹³C-NMR (400 MHz, DMSO-*d*₆) 55.6, 73.4, 92.1, 111.0, 127.2, 127.6, 133.1, 133.5, 133.6, 139.4, 151.9, 152.3, 168.4, 189.1 ppm.

### [Compound 15] 3-((2-chloro-4-formyl-6-methoxyphenoxy)methyl)benzamide

5-chlorovanillin (0.40 g, 2.14 mmol) and 3-(chloromethyl)benzamide (0.36 g, 2.14 mmol) were used to obtain Compound 15 as a white solid (0.32 g, 45.0%).

R_{f} 0.10 (ethyl acetate:n-hexane = 1:1); ¹H-NMR (CDCl₃, 400 MHz) δ 3.88 (s, 3H), 5.11 (s, 2H), 7.30 (d, *J* = 2.0 Hz, 1H), 7.37 (dd, *J* = 7.6, 7.6 Hz, 1H), 7.44 (d, *J* = 2.0 Hz, 1H), 7.59 (d, *J* = 8.0 Hz, 1H), 7.77 (ddd, *J* = 7.6, 1.6, 1.2 Hz, 1H), 794 (dd, *J* = 1.6, 1.6 Hz, 1H), 9.78 (s, 1H); ); ¹³C-NMR (400 MHz, DMSO-*d*₆) 56.4, 74.1, 110.9, 124.1, 127.2, 127.6, 127.9, 128.2, 131.1, 132.6, 134.4, 136.6, 148.2, 154.0, 167.6, 191.1 ppm.

### [Compound 16] 3-((2-bromo-4-formyl-6-methoxyphenoxy)methyl)benzamide

5-bromovanillin (0.18 g, 0.79 mmol) and 3-(chloromethyl)benzamide (0.13 g, 0.79 mmol) were used to obtain Compound 16 as a white solid (0.20 g, 73.2%).

R_{f} 0.16 (ethyl acetate:n-hexane = 1:1); ¹H-NMR (DMSO-*d*₆, 400 MHz) δ 3.88 (s, 3H), 5.10 (s, 2H), 7.34 (d, *J* = 2.0 Hz, 1H), 7.38 (dd, *J* = 8.0, 8.0 Hz, 1H), 7.60 (d, *J* = 2.0 Hz, 1H), 7.62 (ddd, *J* = 7.6, 1.2, 1.2 Hz 1H), 7.78 (ddd, *J* = 7.6, 1.2, 1.2 Hz 1H), 7.96 (dd, *J* = 1.2, 1.2 Hz, 1H), 9.78 (s, 1H); ¹³C-NMR (400 MHz, DMSO-*d*₆) 55.7, 73.8, 110.0, 117.7, 126.9, 127.2, 127.6, 127.9, 131.0, 132.6, 133.5, 136.3, 149.5, 153.6, 168.5, 189.3 ppm.

### [Compound 17] 3-((4-formyl-2-iodo-6-methoxyphenoxy)methyl)benzamide

5-iodovanillin (0.26 g, 0.94 mmol) and 3-(chloromethyl)benzamide (0.16 g, 0.94 mmol) were used to obtain Compound 17 as a white solid (0.28 g, 72.4%).

R_{f} 0.10 (ethyl acetate:n-hexane = 1:1); ¹H-NMR (DMSO-*d*₆, 400 MHz) δ 3.88 (s, 3H), 5.09 (s, 2H), 7.36 (d, *J* = 2.0 Hz, 1H), 7.38 (dd, *J* = 8.0, 8.0 Hz, 1H), 7.65 (ddd, *J* = 7.6, 1.6, 1.6 Hz 1H), 7.78 (ddd, *J* = 7.6, 1.2, 1.2, Hz 1H), 7.79 (d, *J* = 2.0 Hz, 1H), 7.98 (dd, *J* = 1.6, 1.6 Hz, 1H), 9.76 (s, 1H); ¹³C-NMR (400 MHz, DMSO-*d*₆) 55.6, 73.6, 92.2, 111.0, 126.9, 127.3, 127.8, 131.1, 133.5, 113.6, 133.7, 136.2, 151.9, 152.4, 168.5, 189.1 ppm.

### [Synthesis Method 2] Synthesis Examples 1 to 24(A20 to A43)

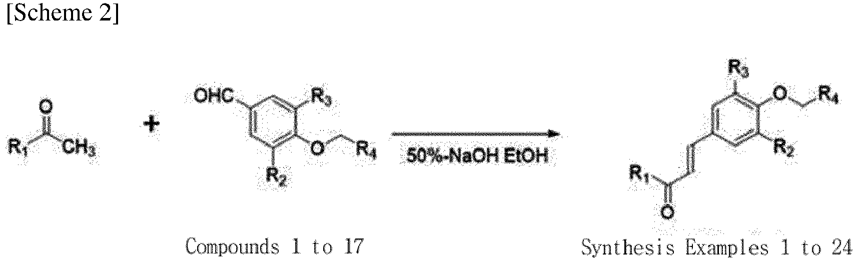

The substituted O-benzylated benzaldehyde derivative was mixed with an acetophenone derivative using 10 mL of ethanol as a solvent, and 50% NaOH (2.0 to 5.0 equivalents) was added thereto. Thereafter, the mixture was stirred at room temperature for 24 hours, and water was further added thereto. Then, the mixture was extracted using ethyl acetate, and the extract was washed with water, NaHCO₃, and brine, and then dried over MgSO₄. Then, the solvent was completely removed under reduced pressure, and silica gel chromatography was then performed to obtain Synthesis Examples 1 to 24 (see Table 3).

**[Table 3]**

| Synthesis Example | R₁ | R₂ | R₃ | R₄ |
|---|---|---|---|---|
| 1 (A20) | 3-aminophenyl | OCH₃ | Cl | 3-carbamoylphenyl |
| 2 (A21) | 3-aminophenyl | OCH₃ | Br | 3-carbamoylphenyl |
| 3 (A22) | 3-aminophenyl | OCH₃ | I | 3-carbamoylphenyl |
| 4 (A23) | 3-carbamoylphenyl | OCH₃ | Cl | 3-carbamoylphenyl |
| 5 (A24) | 3-carbamoylphenyl | OCH₃ | Br | 3-carbamoylphenyl |
| 6 (A25) | 3-carbamoylphenyl | OCH₃ | I | 3-carbamoylphenyl |
| 7 (A26) | 3-hydroxyphenyl | OCH₃ | Cl | 3-carbamoylphenyl |
| 8 (A27) | 3-hydroxyphenyl | OCH₃ | Br | 3-carbamoylphenyl |
| 9 (A28) | 3-hydroxyphenyl | OCH₃ | I | 3-carbamoylphenyl |
| 10 (A29) | 3,4,5-trimethoxyphenyl | OCH₃ | Cl | 3-chlorophenyl |
| 11 (A30) | 3,4,5-trimethoxyphenyl | OCH₃ | Br | 3-chlorophenyl |
| 12 (A31) | 3,4,5-trimethoxyphenyl | OCH₃ | I | 3-chlorophenyl |
| 13 (A32) | 3,4,5-trimethoxyphenyl | OCH₃ | Cl | 3-carbamoylphenyl |
| 14 (A33) | 3,4,5-trimethoxyphenyl | OCH₃ | Br | 3-carbamoylphenyl |
| 15 (A34) | 3,4,5-trimethoxyphenyl | OCH₃ | I | 3-carbamoylphenyl |
| 16 (A35) | 3-hydroxyphenyl | OCH₃ | Cl | 3-chlorophenyl |
| 17 (A36) | 3-hydroxyphenyl | OCH₃ | Br | 3-chlorophenyl |
| 18 (A37) | 3-hydroxyphenyl | OCH₃ | I | 3-chlorophenyl |
| 19 (A38) | 3-aminophenyl(3-aminophenyl) | OCH₃ | Cl | 3-chlorophenyl |
| 20 (A39) | 3-aminophenyl | OCH₃ | Br | 3-chlorophenyl |
| 21 (A40) | 3-aminophenyl | OCH₃ | I | 3-chlorophenyl |
| 22 (A41) | 3-carbamoylphenyl | OCH₃ | Cl | 3-chlorophenyl |
| 23 (A42) | 3-carbamoylphenyl | OCH₃ | Br | 3-chlorophenyl |
| 24 (A43) | 3-carbamoylphenyl | OCH₃ | I | 3-chlorophenyl |

### [Synthesis Example 1] (E)-3-((4-(3-(3-aminophenyl)-3-oxoprop-1-en-1-yl)-2-chloro-6-methoxyphenoxy)methyl) benzamide (A20)

This synthesis was performed using Compound 15 (200 mg, 0.63 mmol) and 3-aminoacetophenone (85 mg, 0.63 mmol), and the resulting product was extracted by silica gel column chromatography (elution: ethyl acetate:dichloromethane = 4:1) to obtain Synthesis Example 1 (A20) as a yellow solid (78 mg, 28.6%).

R_{f} 0.42 (ethyl acetate:dichloromethane = 4:1); ¹H-NMR (400MHz, DMSO-*d*₆) δ 3.95 (s, 3H), 5.11 (s, 2H), 5.36 (s, 2H), 6.86 (ddd, *J* = 7.6, 2.0, 0.8 Hz, 1H), 7.21 (dd, *J* = 8.0, 7.6 Hz, 1H), 7.27 (dd, *J* = 2.0, 2.0 Hz, 1H), 7.37-7.39 (m, 2H), 7.47 (dd, J = 8.0, 7.6 Hz, 1H), 7.56 (d, *J* = 1.6 Hz, 1H), 7.62 (d, *J* = 1.6 Hz, 1H), 7.63 (d, *J* = 15.6 Hz, 1H), 7.64 (d, *J* = 8.0 Hz, 1H), 7.83 (d, *J* = 15.6 Hz, 1H), 7.86 (ddd, *J* = 7.6, 1.6, 1.2 Hz, 1H), 8.00 (br s, 2H).

### [Synthesis Example 2] (E)-3-((4-(3-(3-aminophenyl)-3-oxoprop-1-en-1-yl)-2-bromo-6-methoxyphenoxy)methyl) benzamide (A21)

This synthesis was performed using Compound 16 (150 mg, 0.41 mmol) and 3-aminoacetophenone (49 mg, 0.36 mmol), and the resulting product was extracted by silica gel column chromatography (elution: ethyl acetate:dichloromethane = 4:1) to obtain Synthesis Example 2 (A21) as a yellow solid (35 mg, 20.1%).

R_{f} 0.29 (ethyl acetate:dichloromethane = 4:1); ¹H-NMR (400MHz, DMSO-*d*₆) δ 3.95 (s, 3H), 5.09 (s, 2H), 5.36 (s, 2H), 6.85 (ddd, *J* = 8.0, 2.4, 1.2 Hz, 1H), 7.21 (dd, *J* = 8.0, 7.6 Hz, 1H), 7.26 (dd, *J* = 2.0, 2.0 Hz, 1H), 7.37-7.39 (m, 2H), 7.48 (dd, *J* = 7.6, 7.6 Hz, 1H), 7.60 (d, *J* = 1.6 Hz, 1H), 7.62 (d, *J* = 16.0 Hz, 1H), 7.67 (d, *J* = 8.0 Hz, 1H), 7.75 (d, *J* = 1.6 Hz, 1H), 7.82 (d, *J* = 15.6 Hz, 1H), 7.85 (ddd, *J* = 8.0, 1.2, 1.2 Hz, 1H), 8.00 (br s, 2H); ¹³C-NMR (100 MHz, DMSO-*d*₆) 51.7, 69.1, 107.8, 108.2, 111.8, 112.7, 114.0, 118.3, 120.2, 122.4, 122.8, 123.4, 124.4, 126.3, 127.7, 129.6, 132.1, 133.6, 137.0, 141.1, 144.4, 148.8, 162.9, 184.7 ppm.

### [Synthesis Example 3] (E)-3-((4-(3-(3-aminophenyl)-3-oxoprop-1-en-1-yl)-2-iodo-6-methoxyphenoxy)methyl) benzamide (A22)

This synthesis was performed using Compound 17 (150 mg, 0.36 mmol) and 3-aminoacetophenone (49 mg, 0.36 mmol), and the resulting product was extracted by silica gel column chromatography (elution: ethyl acetate:dichloromethane = 4:1) to obtain Synthesis Example 3 (A22) as a yellow solid (13 mg, 6.8%).

R_{f} 0.38 (ethyl acetate:dichloromethane = 4:1); ¹H-NMR (400MHz, DMSO-*d*₆) δ 3.94 (s, 3H), 5.06 (s, 2H), 5.35 (s, 2H), 6.85 (ddd, *J* = 7.6, 2.0, 0.8 Hz, 1H), 7.21 (dd, *J* = 7.6, 7.6 Hz, 1H), 7.26 (dd, *J* = 2.0, 2.0 Hz, 1H), 7.36-7.39 (m, 2H), 7.49 (dd, *J* = 7.6, 7.6 Hz, 1H), 7.60 (d, *J* = 16.0 Hz, 1H), 7.61 (d, *J* = 1.6 Hz, 1H), 7.70 (d, *J* = 8.0 Hz, 1H), 7.80 (d, *J* = 15.6 Hz, 1H), 7.85 (ddd, *J* = 8.0, 1.6, 1.2 Hz, 1H), 7.90 (d, *J* = 1.6 Hz, 1H), 8.06 (br s, 2H).

### [Synthesis Example 4] (E)-3-(3-(4-((3-carbamoylbenzyl)oxy)-3-chloro-5-methoxyphenyl)acryloyl) benzamide (A23)

This synthesis was performed using Compound 15 (100 mg, 0.31 mmol) and 3-carbamoylacetophenone (51 mg, 0.31 mmol), and the resulting product was extracted by silica gel column chromatography (elution: methanol:dichloromethane = 1:10) to obtain Synthesis Example 4 (A23) as a pale yellow solid (60 mg, 41.3%).

R_{f} 0.50 (methanol:dichloromethane = 1:9); ¹H-NMR (400MHz, DMSO-*d*₆) δ 3.89 (s, 3H), 5.06 (s, 2H), 7.12 (d, *J* = 2.0 Hz, 1H), 7.32 (d, *J* = 1.6 Hz, 1H), 7.37 (dd, *J* = 8.0, 7.6 Hz, 1H), 7.52 (dd, *J* = 8.0, 7.6 Hz, 1H), 7.57 (d, *J* = 16.0 Hz, 1H), 7.61 (d, *J* = 8.0, 1.6 Hz, 1H), 7.63 (d, *J* = 16.0 Hz, 1H), 7.78 (d, *J* = 8.0, 1.6, 1.2 Hz, 1H), 7.95 (dd, *J* = 2.0, 1.2 Hz, 1H), 8.09-8.13 (m, 2H), 8.55 (dd, *J* = 1.6, 1.6 Hz, 1H); ¹³C-NMR (100 MHz, DMSO-*d*₆) 56.5, 74.0, 112.5, 122.1, 122.6, 127.1, 127.3, 127.6, 127.7, 128.2, 128.9, 131.1, 131.2, 131.6, 132.0, 134.3, 134.9, 136.9, 137.5, 143.0, 145.0, 153.8, 167.2, 167.7, 188.8 ppm.

### [Synthesis Example 5] (E)-3-(3-(3-bromo-4-((3-carbamoylbenzyl)oxy)-5-methoxyphenyl)acryloyl) benzamide (A24)

This synthesis was performed using Compound 16 (100 mg, 0.27 mmol) and 3-carbamoylacetophenone (45 mg, 0.31 mmol), and the resulting product was extracted by silica gel column chromatography (elution: methanol:dichloromethane = 1:10) to obtain Synthesis Example 5 (A24) as a pale yellow solid (40 mg, 28.5%).

R_{f} 0.50 (methanol:dichloromethane = 1:9); ¹H-NMR (400MHz, DMSO-*d*₆) δ 3.89 (s, 3H), 5.05 (s, 2H), 7.15 (d, *J* = 2.0 Hz, 1H), 7.38 (dd, *J* = 8.0, 7.6 Hz, 1H), 7.47 (d, *J* = 1.6 Hz, 1H), 7.52 (dd, *J* = 8.0, 7.6 Hz, 1H), 7.58 (d, *J* = 15.6 Hz, 1H), 7.61 (d, *J* = 8.0, 1.6 Hz, 1H), 7.63 (d, *J* = 15.6 Hz, 1H), 7.64 (d, *J* = 7.6, 2.0, 1.2 Hz, 1H), 7.78 (ddd, *J* = 8.0, 1.6, 1.2 Hz, 1H), 7.97 (dd, *J* = 2.0, 2.0 Hz, 1H), 8.09-8.13 (m, 2H), 8.55 (dd, *J* = 1.6, 1.6 Hz, 1H); ¹³C-NMR (100 MHz, DMSO-*d*₆) 56.5, 73.9, 113.1, 117.5, 122.6, 125.0, 127.1, 127.4, 127.6, 128.2, 128.9, 131.1, 131.2, 132.0, 132.3, 134.3, 134.9, 136.9, 137.6, 142.9, 146.1, 153.6, 167.2, 167.7, 188.8 ppm.

### [Synthesis Example 6] (E)-3-(3-(4-((3-carbamoylbenzyl)oxy)-3-iodo-5-methoxyphenyl)acryloyl) benzamide (A25)

This synthesis was performed using Compound 17 (100 mg, 0.24 mmol) and 3-carbamoylacetophenone (40 mg, 0.24 mmol), and the resulting product was extracted by silica gel column chromatography (elution: methanol:dichloromethane = 1:10) to obtain Synthesis Example 6 (A25) as a pale yellow solid (35 mg, 25.9%).

R_{f} 0.50 (methanol:dichloromethane = 1:9); ¹H-NMR (400MHz, DMSO-d₆) δ 3.94 (s, 3H), 5.05 (s, 2H), 7.42 (dd, *J* = 7.6, 7.6 Hz, 1H), 7.48 (d, *J* = 2.0 Hz, 1H), 7.58 (dd, *J* = 7.6, 7.6 Hz, 1H), 7.65 (d, *J* = 15.6 Hz, 1H), 7.67 (dd, *J* = 8.0, 2.0 Hz, 1H), 7.82 (d, *J* = 2.0 Hz, 1H), 7.83 (ddd, *J* = 7.6, 1.6, 1.2 Hz, 1H), 7.84 (d, *J* = 15.6 Hz, 1H), 8.01 (dd, *J* = 2.0, 1.6 Hz, 1H), 8.12-8.15 (m, 1H), 8.21 (ddd, *J* = 8.0, 1.6, 1.2 Hz, 1H), 8.58 (ddd, *J* = 1.6, 1.6 Hz, 1H); ¹³C-NMR (100 MHz, DMSO-*d*₆) 56.3, 73.6, 93.7, 113.8, 122.3, 127.1, 127.3, 127.6, 128.2, 128.9, 130.8, 131.1, 131.2, 132.0, 133.1, 134.3, 134.8, 136.9, 137.6, 142.8, 148.7, 152.4, 167.2, 167.7, 188.8 ppm.

### [Synthesis Example 7] (E)-3-((2-chloro-4-(3-(3-hydroxyphenyl)-3-oxoprop-1-en-1-yl)-6-methoxyphenoxy) methyl) benzamide (A26)

This synthesis was performed using Compound 15 (200 mg, 0.63 mmol) and 1-(2-((tetrahydro-2H-pyran-2-yl)oxy)phenyl)ethan-1-one (138 mg, 0.63 mmol), and the resulting product was extracted by silica gel column chromatography (elution: ethyl acetate:dichloromethane = 4:1) to obtain Synthesis Example 7 (A26) as a yellow solid (35 mg, 12.8%).

R_{f} 0.45 (ethyl acetate:dichloromethane = 4:1); ¹H-NMR (400MHz, DMSO-*d*₆) δ 3.96 (s, 3H), 5.11 (s, 2H), 7.07 (ddd, *J* = 8.0, 2.4, 0.8 Hz, 1H), 7.36-7.40 (m, 2H),7.47 (dd, *J* = 8.0, 7.6 Hz, 1H), 7.49 (dd, *J* = 1.6, 1.6 Hz, 1H), 7.59 (d, *J* = 16.0 Hz, 1H), 7.63-7.67 (m, 4H), 7.85 (ddd, *J* = 8.0, 1.6, 1.2 Hz, 1H), 7.90 (d, *J* = 15.6 Hz, 1H), 7.98-8.01 (m, 2H); ¹³C-NMR (100 MHz, DMSO-*d*₆) 56.5, 74.0, 112.0, 114.7, 119.7, 120.4, 122.3, 122.8, 127.1, 127.5, 127.6, 128.2, 129.8, 131.1, 131.7, 134.3, 136.9, 138.9, 142.4, 144.9, 153.8, 157.7, 167.7, 189.0 ppm.

### [Synthesis Example 8] (E)-3-((2-bromo-4-(3-(3-hydroxyphenyl)-3-oxoprop-1-en-1-yl)-6-methoxyphenoxy) methyl) benzamide (A27)

This synthesis was performed using Compound 16 (100 mg, 0.27 mmol) and 1-(2-((tetrahydro-2H-pyran-2-yl)oxy)phenyl)ethan-1-one (60 mg, 0.27 mmol), and the resulting product was extracted by silica gel column chromatography (elution: methanol:dichloromethane = 1:10) to obtain Synthesis Example 8 (A27) as a pale yellow solid (38 mg, 28.9%).

R_{f} 0.56 (methanol:dichloromethane = 1:9); ¹H-NMR (400MHz, DMSO-d₆) δ 3.96 (s, 3H), 5.09 (s, 2H), 7.07 (ddd, *J* = 7.6, 2.0, 1.2 Hz, 1H), 7.38 (dd, *J* = 8.0, 8.0 Hz, 1H), 7.39 (br s, 1H), 7.48 (dd, *J* = 8.0, 7.6 Hz, 1H), 7.49 (d, *J* = 2.4 Hz, 1H), 7.63 (d, *J* = 2.0 Hz, 1H), 7.65 (d, *J* = 15.6 Hz, 1H), 7.67 (dd, *J* = 7.6, 1.6 Hz, 1H), 7.79 (d, *J* = 1.6 Hz, 1H), 7.85 (ddd, *J* = 7.6, 1.6, 1.2 Hz, 1H), 7.89 (d, *J* = 15.6 Hz, 1H), 8.00 (br s, 1H); ¹³C-NMR (100 MHz, DMSO-d₆) 56.5, 73.9, 112.6, 114.7, 117.4, 119.7, 120.3, 122.8, 125.1, 127.1, 127.6, 128.2, 129.8, 131.1, 132.4, 134.3, 136.9, 138.9, 142.3, 145.9, 153.6, 157.7, 167.7, 188.9 ppm.

### [Synthesis Example 9] (E)-3-((4-(3-(3-hydroxyphenyl)-3-oxoprop-1-en-1-yl)-2-iodo-6-methoxyphenoxy) methyl) benzamide (A28)

This synthesis was performed using Compound 17 (200 mg, 0.49 mmol) and 1-(2-((tetrahydro-2H-pyran-2-yl)oxy)phenyl)ethan-1-one (107 mg, 0.49 mmol), and the resulting product was extracted by silica gel column chromatography (elution: methanol:dichloromethane = 1:20) to obtain Synthesis Example 9 (A28) as a yellow solid (136 mg, 52.9%).

R_{f} 0.53 (methanol:dichloromethane = 1:9); ¹H-NMR (400MHz, DMSO-*d*₆) δ 3.87 (s, 3H), 5.02 (s, 2H), 7.00 (ddd, *J* = 7.6, 2.0, 1.2 Hz, 1H), 7.13 (d, *J* = 1.6 Hz, 1H), 7.22 (dd, *J* = 8.0, 8.0 Hz, 1H), 7.35-7.41 (m, 4H), 7.53 (d, *J* = 15.6 Hz, 1H), 7.59 (d, *J* = 2.0 Hz, 1H), 7.66 (d, *J* = 7.6 Hz, 1H), 7.78 (dd, *J* = 8.0, 1.6 Hz, 1H), 7.99 (dd, *J* = 1.6, 1.6 Hz, 1H)).

### [Synthesis Example 10] (E)-3-(3-chloro-4-((3-chlorobenzyl)oxy)-5-methoxyphenyl)-1-(3,4,5-trimethoxyphenyl) prop-2-en-1-one (A29)

This synthesis was performed using Compound 1 (100 mg, 0.32 mmol) and 3,4,5-trimethoxyacetophenone (67.4 mg, 0.32 mmol), and the resulting product was extracted by silica gel column chromatography (elution: ethyl acetate:n-hexane = 1:3) to obtain Synthesis Example 10 (A29) as an off-white solid (30 mg, 18.6%).

R_{f} 0.19 (ethyl acetate:n-hexane = 1:3); ¹H-NMR (400MHz, CDCl₃) δ 3.93 (s, 3H), 3.95 (s, 3H), 3.96 (s, 6H), 5.07 (s, 2H), 7.04 (d, *J* = 2.0 Hz, 1H), 7.26 (d, *J* = 2.4 Hz, 2H), 7.30-7.32 (m, 2H), 7.34 (d, *J* = 2.0 Hz, 1H), 7.37 (d, *J* = 15.6 Hz, 1H), 7.37-7.39 (m, 1H), 7.55 (dd, *J* = 0.8, 0.8 Hz, 1H), 7.68 (d, *J* = 15.6 Hz, 1H); ¹³C-NMR (100 MHz, CDCl₃) 56.5, 56.7, 61.2, 74.4, 106.1, 106.5, 111.5, 121.9, 122.4, 126.5, 128.5, 128.6, 129.4, 129.9, 131.9, 133.5, 134.5, 139.0, 143.3, 146.0, 153.4, 154.2, 189.1 ppm.

### [Synthesis Example 11] (E)-3-(3-bromo-4-((3-chlorobenzyl)oxy)-5-methoxyphenyl)-1-(3,4,5-trimethoxyphenyl) prop-2-en-1-one (A30)

This synthesis was performed using Compound 2 (100 mg, 0.28 mmol) and 3,4,5-trimethoxyacetophenone (59 mg, 0.28 mmol), and the resulting product was extracted by silica gel column chromatography (elution: ethyl acetate: n-hexane = 1:3) to obtain Synthesis Example 11 (A30) as a yellow solid (55 mg, 35.8%).

R_{f} 0.26 (ethyl acetate: n-hexane = 1:3); ¹H-NMR (400MHz, CDCl₃) δ 3.94 (s, 3H), 3.95 (s, 3H), 3.96 (s, 6H), 5.06 (s, 2H), 7.08 (d, *J* = 2.0 Hz, 1H), 7.26 (d, *J* = 2.4 Hz, 2H), 7.31-7.34 (m, 2H), 7.36 (d, *J* = 15.6 Hz, 1H), 7.41 (ddd, *J* = 8.4, 1.2, 1.2 Hz, 1H), 7.51 (d, *J* = 1.6 Hz, 1H), 7.57 (br s, 1H), 7.68 (d, *J* = 15.6 Hz, 1H); ¹³C-NMR (100 MHz, DMSO-*d*₆) 56.3, 56.5, 60.2, 73.2, 106.4, 113.4, 117.4, 122.6, 124.6, 126.7, 127.9, 128.0, 130.2, 132.4, 132.9, 139.3, 142.1, 142.3, 145.7, 152.9, 153.4, 187.9 ppm.

### [Synthesis Example 12] (E)-3-(4-((3-chlorobenzyl)oxy)-3-iodo-5-methoxyphenyl)-1-(3,4,5-trimethoxyphenyl) prop-2-en-1-one (A31)

This synthesis was performed using Compound 3 (100 mg, 0.25 mmol) and 3,4,5-trimethoxyacetophenone (52 mg, 0.25 mmol), and the resulting product was extracted by silica gel column chromatography (elution: ethyl acetate:n-hexane = 1:3) to obtain Synthesis Example 12 (A31) as a pale yellow solid (57 mg, 38.7%).

R_{f} 0.23 (ethyl acetate:n-hexane = 1:3); ¹H-NMR (400MHz, CDCl₃) δ 3.93 (s, 3H), 3.95 (s, 3H), 3.96 (s, 6H), 5.05 (s, 2H), 7.12 (d, *J* = 2.0 Hz, 1H), 7.26 (d, *J* = 2.0 Hz, 2H), 7.32-7.34 (m, 2H), 7.35 (d, *J* = 15.6 Hz, 1H), 7.44 (ddd, *J* = 7.2, 1.6, 1.2 Hz, 1H), 7.59 (dd, *J* = 1.2, 1.2 Hz, 1H), 7.67 (d, *J* = 15.6 Hz, 1H), 7.71 (d, *J* = 2.0 Hz, 1H); ¹³C-NMR (100 MHz, DMSO-d₆) 56.3, 56.4, 60.2, 72.9, 93.6, 106.4, 114.0, 122.4, 126.7, 127.9, 130.2, 130.6, 132.8, 132.9, 133.2, 139.3, 142.1, 142.2, 148.4, 152.3, 152.9, 187.9 ppm.

### [Synthesis Example 13] (E)-3-((2-chloro-6-methoxy-4-(3-oxo-3-(3,4,5-trimethoxyphenyl)prop-1-en-1-yl)phenoxy) methyl)benzamide (A32)

This synthesis was performed using Compound 15 (170 mg, 0.53 mmol) and 3,4,5-trimethoxyacetophenone (112 mg, 0.53 mmol), and the resulting product was extracted by silica gel column chromatography (elution: ethyl acetate:n-hexane = 2:1) to obtain Synthesis Example 13 (A32) as a pale yellow solid (140 mg, 51.3%).

R_{f} 0.13 (ethyl acetate:n-hexane = 2:1); ¹H-NMR (400MHz, DMSO-*d*₆) δ 3.84 (s, 3H), 3.87 (s, 3H), 3.88 (s, 6H), 5.05 (s, 2H), 7.06 (d, *J* = 1.6 Hz, 1H), 7.22 (s, 2H), 7.29 (d, *J* = 2.0 Hz, 1H), 7.38 (dd, *J* = 7.6, 7.6 Hz, 1H), 7.39 (d, *J* = 15.6 Hz, 1H), 7.58 (d, *J* = 15.6 Hz, 1H), 7.60 (dd, *J* = 7.6, 7.6 Hz, 1H), 7.77 (ddd, *J* = 7.6, 1.6, 1.2 Hz, 1H), 7.95 (dd, *J* = 1.6, 1.2 Hz, 1H); ¹³C-NMR (100 MHz, DMSO-*d*6) 56.0, 56.1, 60.5, 74.1, 105.9, 110.9, 121.5, 122.0, 127.1, 127.4, 128.1, 128.5, 131.2, 131.3, 133.0, 133.6, 136.8, 142.2, 142.6, 145.3, 152.8, 153.7, 168.8, 188.4 ppm.

### [Synthesis Example 14] (E)-3-((2-bromo-6-methoxy-4-(3-oxo-3-(3,4,5-trimethoxyphenyl)prop-1-en-1-yl)phenoxy) methyl)benzamide (A33)

This synthesis was performed using Compound 16 (100 mg, 0.27 mmol) and 3,4,5-trimethoxyacetophenone (58 mg, 0.27 mmol), and the resulting product was extracted to obtain Synthesis Example 14 (A33) as a pale yellow solid (100 mg, 65.4%).

R_{f} 0.13 (ethyl acetate:n-hexane = 2:1); ¹H-NMR (400MHz, CDCl₃) δ 3.84 (s, 3H), 3.88 (s, 9H), 5.04 (s, 2H), 7.11 (d, *J* = 1.6 Hz, 1H), 7.22 (s, 2H), 7.29 (d, *J* = 2.0 Hz, 1H), 7.38 (dd, *J* = 8.0, 8.0 Hz, 1H), 7.40 (d, *J* = 15.6 Hz, 1H), 7.44 (d, *J* = 1.6 Hz, 1H), 7.58 (d, *J* = 15.6 Hz, 1H), 7.63 (d, *J* = 8.0 Hz, 1H), 7.78 (ddd, *J* = 7.6, 1.6, 1.2 Hz, 1H), 7.97 (dd, *J* = 1.6, 1.2 Hz, 1H); ¹³C-NMR (100 MHz, DMSO-*d*₆) 55.7, 55.9, 60.3, 73.8, 105.7, 111.3, 117.7, 121.8, 124.2, 126.8, 127.2, 127.8, 131.0, 131.7, 132.7, 133.4, 136.6, 141.9, 142.2, 146.1, 152.5, 153.3, 168.6, 188.1 ppm.

### [Synthesis Example 15] (E)-3-((2-iodo-6-methoxy-4-(3-oxo-3-(3,4,5-trimethoxyphenyl)prop-1-en-1-yl)phenoxy) methyl)benzamide (A34)

This synthesis was performed using Compound 17 (100 mg, 0.24 mmol) and 3,4,5-trimethoxyacetophenone (51 mg, 0.24 mmol), and the resulting product was extracted to obtain Synthesis Example 15 (A34) as a pale yellow solid (75 mg, 51.2%).

R_{f} 0.13 (ethyl acetate:n-hexane = 2:1); ¹H-NMR (400MHz, CDCl₃) δ 3.84 (s, 3H), 3.87 (s, 3H), 3.88 (s, 6H), 5.03 (s, 2H), 7.14 (d, *J* = 1.6 Hz, 1H), 7.21 (s, 2H), 7.38 (d, *J* = 15.2 Hz, 1H), 7.39 (dd, *J* = 7.6, 7.6 Hz, 1H), 7.57 (d, *J* = 15.6 Hz, 1H), 7.63 (d, *J* = 1.6 Hz, 1H), 7.67 (d, *J* = 7.6 Hz, 1H), 7.78 (ddd, *J* = 8.0, 1.6, 1.2 Hz, 1H), 7.99 (dd, *J* = 1.6, 1.2 Hz, 1H); ¹³C-NMR (100 MHz, DMSO-*d*₆) 55.6, 55.9, 60.3, 73.6, 92.6, 105.7, 112.3, 121.7, 126.8, 127.3, 127.8, 130.2, 131.1, 132.6, 132.7, 133.5, 136.5, 141.9, 142.1, 148.6, 152.1, 152.5, 168.5, 188.1 ppm.

### [Synthesis Example 16] (E)-3-(3-chloro-4-((3-chlorobenzyl)oxy)-5-methoxyphenyl)-1-(3-hydroxyphenyl)prop-2-en-1-one (A35)

This synthesis was performed using Compound 1 (100 mg, 0.32 mmol) and 1-(3-((tetrahydro-2H-pyran-2-yl)oxy)phenyl)ethan-1-one (70.1 mg, 0.32 mmol), and the resulting product was extracted by silica gel column chromatography (elution: ethyl acetate:n-hexane = 1:3) to obtain Synthesis Example 16 (A35) as an ivory solid (26 mg, 18.9%).

R_{f} 0.13 (ethyl acetate:n-hexane = 1;3); ¹H-NMR (400MHz, DMSO-*d*₆) δ 3.88 (s, 3H), 4.98 (s, 2H), 7.00 (ddd, *J* = 8.0, 1.6, 0.8 Hz, 1H), 7.06 (d, *J* = 2.0 Hz, 1H), 7.22-7.26 (m, 4H),7.30 (ddd, *J* = 8.0, 2.0, 1.6 Hz, 1H), 7.37-7.41 (m, 2H), 7.39 (d, *J* = 15.6 Hz, 1H), 7.46 (d, *J* = *2.4 Hz, 1H*)*, 7.54* (*d, J* = *15.6 Hz, 1H*)*.*

### [Synthesis Example 17] (E)-3-(3-bromo-4-((3-chlorobenzyl)oxy)-5-methoxyphenyl)-1-(3-hydroxyphenyl)prop-2-en-1-one (A36)

This synthesis was performed using Compound 2 (100 mg, 0.28 mmol) and 1-(3-((tetrahydro-2H-pyran-2-yl)oxy)phenyl)ethan-1-one (62 mg, 0.28 mmol), and the resulting product was extracted by silica gel column chromatography (elution: ethyl acetate:n-hexane = 1:3) to obtain Synthesis Example 17 (A36) as an ivory solid (62 mg, 46.5%).

R_{f} 0.14 (ethyl acetate:n-hexane = 1;3); ¹H-NMR (400MHz, DMSO-*d*₆) δ 3.95 (s, 3H), 5.07 (s, 2H), 7.07 (dd, *J* = 8.0, 2.4 Hz, 1H), 7.38 (dd, *J* = 8.0, 7.6 Hz, 1H), 7.41-7.49 (m, 4H), 7.57 (br s, 1H), 7.62 (d, *J* = 2.0 Hz, 1H), 7.64 (d, *J* = 8.0 Hz, 1H), 7.65 (d, *J* = 16.0 Hz, 1H), 7.79 (dd, *J* = 2.0, 1.6 Hz, 1H), 7.90 (d, *J* = 15.6 Hz, 1H), 9.81 (s, 1H); ¹³C-NMR (100 MHz, DMSO-d₆) 56.5, 73.2, 112.6, 114.7, 117.3, 119.7, 120.3, 122.8, 125.1, 126.7, 127.9, 128.0, 129.8, 130.2, 132.4, 132.9, 138.9, 139.3, 142.2, 145.8, 153.5, 157.7, 188.9 ppm.

### [Synthesis Example 18] (E)-3-(4-((3-chlorobenzyl)oxy)-3-iodo-5-methoxyphenyl)-1-(3-hydroxyphenyl)prop-2-en-1-one (A37)

This synthesis was performed using Compound 3 (100 mg, 0.25 mmol) and 1-(3-((tetrahydro-2H-pyran-2-yl)oxy)phenyl)ethan-1-one (55 mg, 0.25 mmol), and the resulting product was extracted by silica gel column chromatography (elution: ethyl acetate:n-hexane = 1:3) to obtain Synthesis Example 18 (A37) as a yellow solid (57 mg, 43.8%).

R_{f} 0.15 (ethyl acetate:n-hexane = 1;3); ¹H-NMR (400MHz, CDCl₃) δ 3.84 (s, 3H), 4.94 (s, 2H), 7.00 (ddd, *J* = 8.0, 2.0, 1.2 Hz, 1H), 7.05 (d, *J* = 1.6 Hz, 1H), 7.22-7.26 (m, 3H), 7.31 (d, *J* = 15.6 Hz, 1H), 7.33-7.36 (m, 1H), 7.38-7.41 (m, 2H), 7.50 (br s, 1H), 7.53 (d, *J* = 16.0 Hz, 1H), 7.58 (d, *J* = 2.0 Hz, 1H), 9.02 (s, 1H); ¹³C-NMR (100 MHz, DMSO-*d*₆) 56.3, 72.9, 93.6, 113.3, 114.7, 119.7, 120.3, 122.6, 126.7, 127.9, 128.0, 129.8, 130.2, 131.0, 132.9, 133.2, 138.9, 139.3, 142.2, 148.4, 152.3, 157.7, 188.9 ppm.

### [Synthesis Example 19] (E)-1-(3-aminophenyl)-3-(3-chloro-4-((3-chlorobenzyl)oxy)-5-methoxyphenyl)prop-2-en-1-one (A38)

This synthesis was performed using Compound 1 (230 mg, 0.74 mmol) and 3-aminoacetophenone (100 mg, 0.74 mmol), and the resulting product was extracted by silica gel column chromatography (elution: ethyl acetate:n-hexane = 1:3) to obtain Synthesis Example 19 (A38) as an orange solid (60 mg, 18.9%).

R_{f} 0.06 (ethyl acetate:n-hexane = 1:3); ¹H-NMR (400MHz, CDCl₃) δ 3.72 (s, 3H), 4.82 (s, 2H), 5.36 (s, 2H), 6.91 (d, *J* = 2.0 Hz, 1H), 6.91-6.93 (m, 1H), 7.07-7.14 (m, 5H), 7.22 (d, *J* = 15.6 Hz, 1H), 7.29 (d, *J* = 7.6 Hz, 1H), 7.30 (br s, 1H), 7.33 (dd, *J* = 1.6, 1.6 Hz, 1H), 7.39 (d, *J* = 15.6 Hz, 1H).

### [Synthesis Example 20] (E)-1-(3-aminophenyl)-3-(3-bromo-4-((3-chlorobenzyl)oxy)-5-methoxyphenyl)prop-2-en-1-one (A39)

This synthesis was performed using Compound 2 (100 mg, 0.28 mmol) and 3-aminoacetophenone (38 mg, 0.28 mmol)), and the resulting product was extracted by silica gel column chromatography (elution: ethyl acetate:n-hexane = 1:3) to obtain Synthesis Example 20 (A39) as an orange solid (57 mg, 42.9%).

R_{f} 0.07 (ethyl acetate:n-hexane = 1:3); ¹H-NMR (400MHz, CDCl₃) δ 3.93 (s, 3H), 5.05 (s, 2H), 6.91 (ddd, *J* = 8.0, 1.6, 0.8 Hz, 1H), 7.08 (d, *J* = 1,6 Hz, 1H), 7.29 (dd, *J* = 7.6, 7.6 Hz, 1H), 7.31-7.33 (m, 3H), 7.38 (d, *J* = 15.6 Hz, 1H), 7.37 (dd, *J* = 1.6, 1.2 Hz, 1H), 7.40-7.42 (m, 1H), 7.47 (d, *J* = 1,6 Hz, 1H), 7.57 (br s, 1H), 7.65 (d, *J* = 16.0 Hz, 1H); ¹³C-NMR (100 MHz, DMSO-*d*₆) 56.5, 73.2, 112.5, 112.9, 116.6, 117.3, 118.7, 123.1, 124.9, 126.7, 127.9, 128.0, 129.1, 130.2, 132.5, 132.9, 138.3, 139.3, 141.7, 145.7, 149.1, 153.5, 189.5 ppm.

### [Synthesis Example 21] (E)-1-(3-aminophenyl)-3-(4-((3-chlorobenzyl)oxy)-3-iodo-5-methoxyphenyl)prop-2-en-1-one (A40)

This synthesis was performed using Compound 3 (200 mg, 0.50 mmol) and 3-aminoacetophenone (67 mg, 0.50 mmol), and the resulting product was extracted by silica gel column chromatography (elution: ethyl acetate:n-hexane = 1:3) to obtain Synthesis Example 21 (A40) as a yellow solid (64 mg, 42.9%).

R_{f} 0.08 (ethyl acetate:n-hexane = 1:3); ¹H-NMR (400MHz, DMSO-d₆) δ 3.93 (s, 3H), 5.03 (s, 2H), 5.34 (s, 2H), 6.85 (ddd, *J* = 8.0, 2.8, 0.8, 1H), 7.21 (dd, *J* = 8.0, 7.6 Hz, 1H), 7.26 (dd, *J* = 2.0, 2.0 Hz, 1H), 7.37 (ddd, *J* = 7.6, 1.6, 1.2 Hz, 1H), 7.42-7.46 (m, 2H), 7.48 (ddd, *J* = 7.6, 1.6, 1.2 Hz, 1H), 7.58-7.62 (m, 3H), 7.80 (d, *J* = 16.0 Hz, 1H), 7.90 (d, *J* = 1.6 Hz, 1H); ¹³C-NMR (100 MHz, DMSO-*d*₆) 56.3, 72.9, 93.6, 112.9, 113.3, 116.5, 118.7, 122.9, 126.7, 127.8, 127.9, 129.1, 130.2, 130.7, 132.9, 133.3, 138.3, 139.3, 141.6, 148.3, 149.1, 152.3, 189.4 ppm.

### [Synthesis Example 22] (E)-3-(3-(3-chloro-4-((3-chlorobenzyl)oxy)-5-methoxyphenyl)acryloyl)benzamide (A41)

This synthesis was performed using Compound 1 (100 mg, 0.32 mmol) and 3-carbamoylacetophenone (52 mg, 0.32 mmol), and the resulting product was extracted by silica gel column chromatography (elution: methanol:dichloromethane = 1:10) to obtain Synthesis Example 22 (A41) as a pale yellow solid (41 mg, 28.2%).

R_{f} 0.72 (methanol:dichloromethane = 1:9); ¹H-NMR (400MHz, DMSO-d₆) δ 3.89 (s, 3H), 4.99 (s, 2H), 7.10 (d, *J* = 1.6 Hz, 1H), 7.23 (dd, *J* = 2.4, 2.0 Hz, 1H), 7.25 (dd, *J* = 7.2, 7.2 Hz, 1H), 7.31 (ddd, *J* = 7.2, 2.0, 1.6 Hz, 1H), 7.32 (d, *J* = 2.0 Hz, 1H), 7.46 (br s, 1H), 7.52 (dd, *J* = 8.0, 7.6 Hz, 1H), 7.57 (d, *J* = 15.6 Hz, 1H), 7.63 (d, *J* = 15.6 Hz, 1H), 8.11 (dd, *J* = 7.6, 1.6 Hz, 2H), 8,54 (dd, *J* = 1.6, 1.6 Hz, 1H).

### [Synthesis Example 23] (E)-3-(3-(3-bromo-4-((3-chlorobenzyl)oxy)-5-methoxyphenyl)acryloyl)benzamide (A42)

This synthesis was performed using Compound 3 (100 mg, 0.25 mmol) and 3-carbamoylacetophenone (41 mg, 0.25 mmol), and the resulting product was extracted by silica gel column chromatography (elution: methanol:dichloromethane = 1:10) to obtain Synthesis Example 23 (A42) as a pale yellow solid (51 mg, 37.1%).

R_{f} 0.53 (methanol:dichloromethane = 1:9); ¹H-NMR (400MHz, DMSO-d₆) δ 3.94 (s, 3H), 5.05 (s, 2H), 7.42-7.44 (m, 2H), 7.48 (ddd, *J* = 7.6, 0.8 Hz, 1H), 7.56 (br s, 1H), 7.61 (br s, 1H), 7.64 (d, *J* = 2.0 Hz, 1H), 7.66 (d, *J* = 7.6 Hz, 1H), 7.71 (d, *J* = 15.2 Hz, 1H), 7.96 (d, *J* = 15.2 Hz, 1H), 7.98 (d, *J* = 2.0 Hz, 1H), 8.15 (ddd, *J* = 8.0, 1.2, 1.2 Hz, 1H), 8.20 (br s, 1H), 8.32 (ddd, *J* = 7.6, 1.2, 1.2 Hz, 1H), 8.57 (dd, *J* = 2.0, 1.6 Hz, 1H); ¹³C-NMR (100 MHz, DMSO-d₆) 56.3, 72.9, 93.6, 113.8, 122.4, 126.7, 127.3, 127.9, 128.0, 128.8, 130.2, 130.8, 131.2, 132.0, 132.9, 133.1, 134.8, 137.6, 139.3, 142.8, 148.5, 152.3, 167.2, 188.8 ppm.

### [Synthesis Example 24] (E)-3-(3-(4-((3-chlorobenzyl)oxy)-3-iodo-5-methoxyphenyl)acryloyl)benzamide (A43)

This synthesis was performed using Compound 3 (100 mg, 0.25 mmol) and 3-carbamoylacetophenone (41 mg, 0.25 mmol), and the resulting product was extracted by silica gel column chromatography (elution: methanol:dichloromethane = 1:10) to obtain Synthesis Example 24 (A43) as a pale yellow solid (51 mg, 37.1%).

R_{f} 0.53 (methanol:dichloromethane = 1:9); ¹H-NMR (400MHz, DMSO-d₆) δ 3.94 (s, 3H), 5.05 (s, 2H), 7.42-7.44 (m, 2H), 7.48 (ddd, *J* = 7.6, 0.8 Hz, 1H), 7.56 (br s, 1H), 7.61 (br s, 1H), 7.64 (d, *J* = 2.0 Hz, 1H), 7.66 (d, *J* = 7.6 Hz, 1H), 7.71 (d, *J* = 15.2 Hz, 1H), 7.96 (d, *J* = 15.2 Hz, 1H), 7.98 (d, *J* = 2.0 Hz, 1H), 8.15 (ddd, *J* = 8.0, 1.2, 1.2 Hz, 1H), 8.20 (br s, 1H), 8.32 (ddd, *J* = 7.6, 1.2, 1.2 Hz, 1H), 8.57 (dd, *J* = 2.0, 1.6 Hz, 1H); ¹³C-NMR (100 MHz, DMSO-*d*₆) 56.3, 72.9, 93.6, 113.8, 122.4, 126.7, 127.3, 127.9, 128.0, 128.8, 130.2, 130.8, 131.2, 132.0, 132.9, 133.1, 134.8, 137.6, 139.3, 142.8, 148.5, 152.3, 167.2, 188.8 ppm.

### [Example 4] Confirmation of degree of inhibition of HAT activity of Synthesis Examples 1 to 24

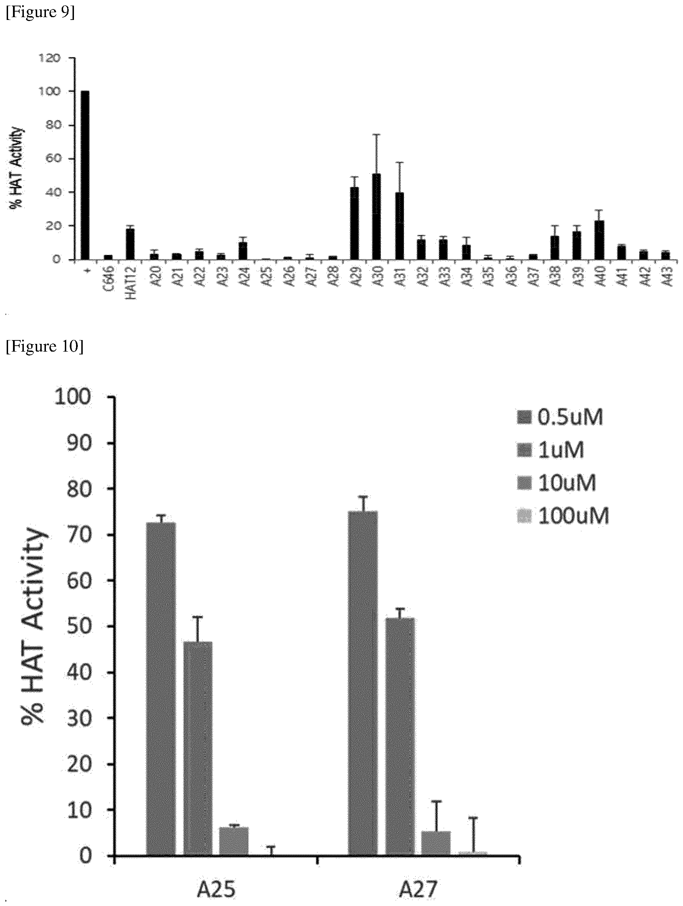

Each of Synthesis Examples 1 to 24 was diluted to 100 µM, and the degree of inhibition of HAT p300 activity was determined in the same manner as in Section [2-1]. The results are shown in FIG. 9. Here, the tissues were treated with C646 and Candidate 12 (HAT-12) as the control instead of Synthesis Examples 1 to 24.

As shown in FIG. 9, it was confirmed that the HAT activity inhibitory effects of Synthesis Examples 1 to 24 (A20 to A43) was 90% or more, which was significantly higher than those of C646 and Candidate 12 corresponding to the control, indicating that the Synthesis Examples 1 to 24 (A20 to A43) inhibited HAT p300 activity. In addition, almost 100% of the HAT p300 activity was inhibited by Synthesis Example 6 (A25) and Synthesis Example 8 (A27).

Based on the results, it can be seen that Synthesis Examples 1 to 24 corresponding to the novel synthetic compounds according to the present invention have a very excellent degree of inhibition of HAT p300 activity. From the results, it can be seen that Synthesis Examples 1 to 24 can be very effectively used to prevent, ameliorate or treat diseases associated with the HAT p300.

### [Example 5] Confirmation of degree of inhibition of HAT p300 activity of Synthesis Example 6 and 8

Synthesis Example 6 and 8 having very excellent HAT p300 inhibitory effects as described in Example 4 were diluted to 0.5 µM, 1 µM, 10 µM, or 100 µM, and the degree of inhibition of HAT p300 activity was determined at each concentration in the same manner as in Section [2-1], and IC₅₀ values were deduced from these results. The results are shown in FIG. 10 and Table 4 below.

**[Table 4]**

| | Synthesis Example 6 (A25) | Synthesis Example 8 (A27) |
|---|---|---|
| IC₅₀ (µM) | 0.87 ± 0.15 | 1.06 ± 0.16 |

As shown in FIG. 10 and Table 4, Synthesis Example 6 and 8 had an IC₅₀ value of 0.87 µM and 1.06 µM, respectively, indicating that Synthesis Example 6 and 8 every effectively inhibited HAT p300 activity.

Based on the results, it can be seen that the novel synthetic compound according to the present invention very effectively inhibited HAT p300 activity because the novel synthetic compound was modified so that additional hydrogen bonds were formed with R1410, T1411, W1466, Y1467 of HAT p300.

### [Example 6] Structural analysis of Candidate 6 (A25) and 8 (A27)

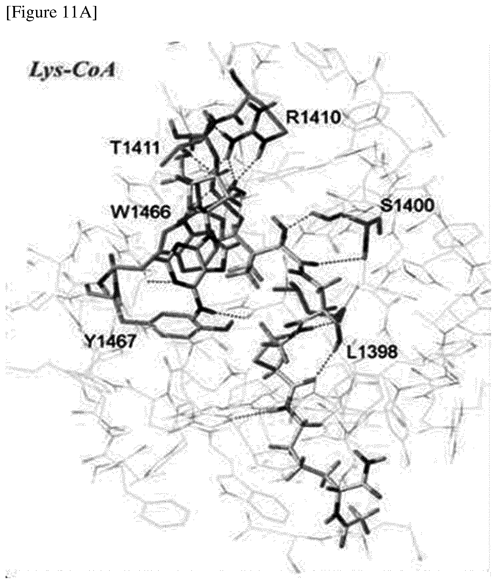

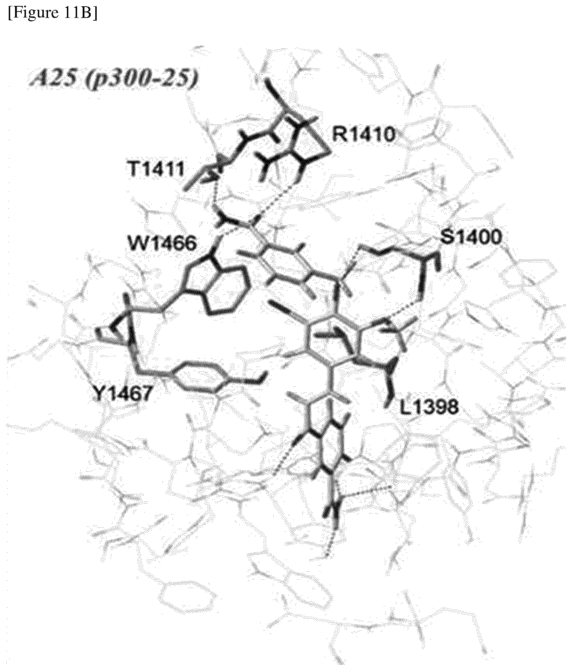

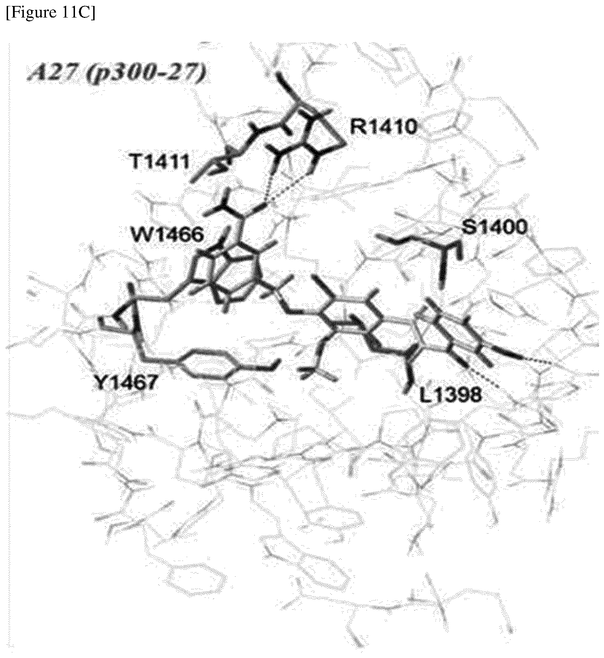

For Candidate 6 (A25) and Candidate 8 (A27) having the best HAT inhibitory effect as described in in Example 5, a molecular docking simulation was performed in the same manner as in Example 3. The results are shown in FIGS. 11A to 11C.

As shown in FIGS. 11A to 11C, it can be seen that, in addition to the hydrogen bonds with S1400 of HAT p300 confirmed from Candidate 12 (HAT-12) in Example 3, the hydrogen bonds with R1410, T1411 and W1466 were further expected in the case of Synthesis Example 6 (A25), and the entire docking pattern was also similar to that of the Lys-CoA. It was expected that the HAT p300 inhibitory activity of Synthesis Example 6 remarkably increased due to such a structure (IC₅₀ = 0.87 ± 0.15 (µM)). In addition, the hydrogen bonds with R1410 and T1411 were further expected in the case of Synthesis Example 8 (A27), and it was expected that the HAT p300 inhibitory activity of Synthesis Example 8 would increase due to such a structure (IC₅₀ = 1.06 ± 0.16 (µM)).

Based on the results, it can be seen that Synthesis Examples 6 and 8 according to the present invention interacted with the HAT p300 domain at a very high level through the additional hydrogen bonding with R1410, T1411 and W1466; or R1410 and T1411 of HAT p300, and thus very effectively inhibited HAT p300 activity.

While the present invention has been described in detail with reference to exemplary embodiments of the present invention, it will be obvious to those of ordinary skill in the art that this specific description is just a preferred embodiment, and is not intended to limit the scope of the present invention. Therefore, it should be understood that the technical scope of the present invention should be defined by the appended claims and equivalents thereof.

### [Industrial Applicability]

The present invention relates to a novel compound which enables additional hydrogen bonding with a specific amino acid position of histone acetyltransferase (HAT) p300 through the structural analysis of the HAT p300. The novel compound of the present invention has an excellent inhibitory effect on HAT p300 activity, and thus can be very effectively used to prevent, ameliorate or treat fibrosis that is a disease associated with the activation of HAT p300.

## Claims

1. A compound selected from a compound represented by the following Formula 1, and a pharmaceutically acceptable salt, an optical isomer, a hydrate, and a solvate thereof: 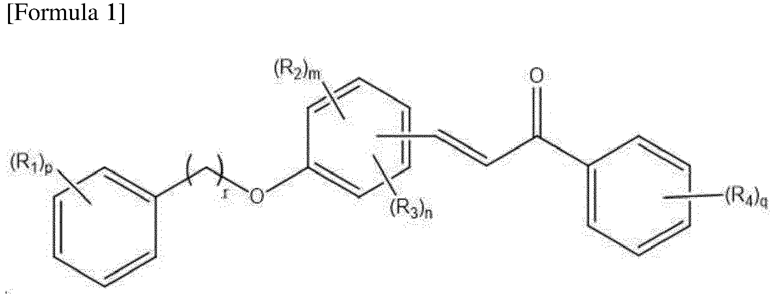 wherein:
p and q are each independently an integer ranging from 1 to 5;
r is an integer ranging from 1 to 4;
m and n are each independently an integer ranging from 1 to 3, provided that m + n is not greater than 4;
R₁ is a carbamoyl group (-C( = O)(NH₂)) or a halogen, wherein when R₁ is present in a plural number, they are the same or different from each other;
R2 and R3 are each independently a C₁-C₆ alkoxy group or a halogen, wherein when each of R2 and R3 is present in a plural number, they are the same or different from each other; and
R4 comprises any one selected from the group consisting of a C₁-C₆ alkoxy group, an amine group (-NH2), a carbamoyl group, and a hydroxyl group (-OH), wherein when R4 is present in a plural number, they are the same or different from each other.

2. The compound of claim 1, wherein p and q are each independently an integer ranging from 1 to 3;
r is an integer of 1 or 2;
m and n are an integer of 1 or 2.

3. The compound of claim 1, wherein R₁ is a carbamoyl group (-C( = O)(NH2)).

4. The compound of claim 1, wherein R₄ comprises any one selected from an amine group, a carbamoyl group, or a hydroxyl group.

5. The compound of claim 1, wherein the compound comprises any one selected from the group consisting of the following compounds:

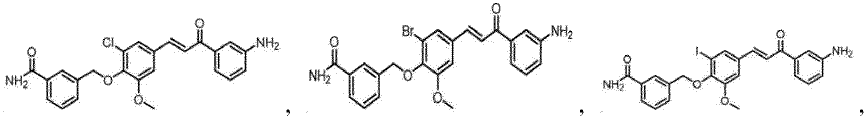

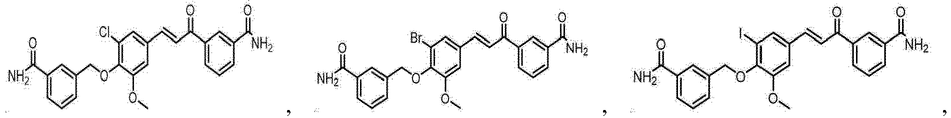

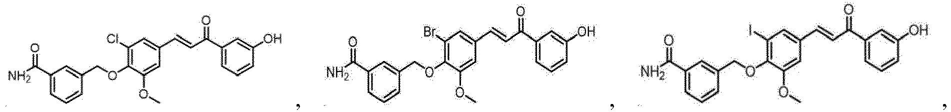

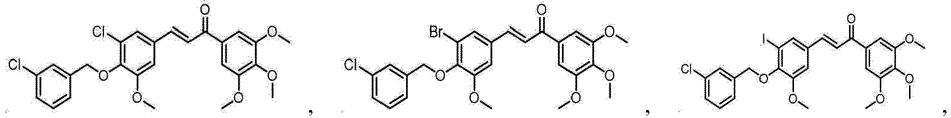

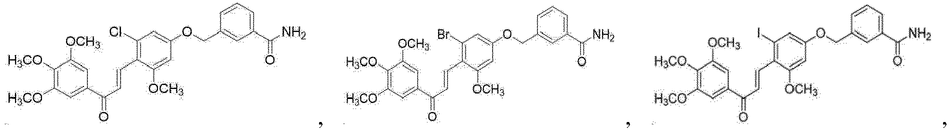

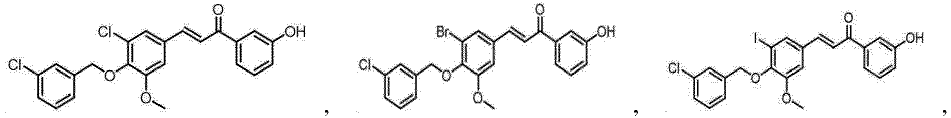

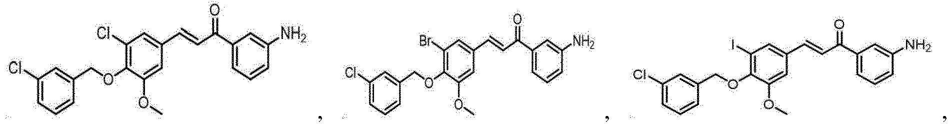

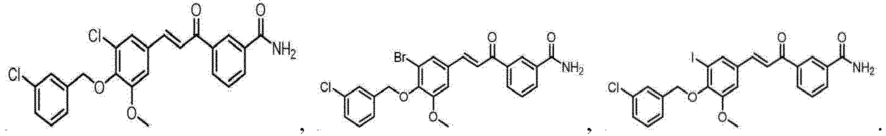

6. A compound for inhibiting histone acetyltransferase p300, which is selected from a compound represented by the following Formula 1, and a pharmaceutically acceptable salt, an optical isomer, a hydrate, and a solvate thereof: 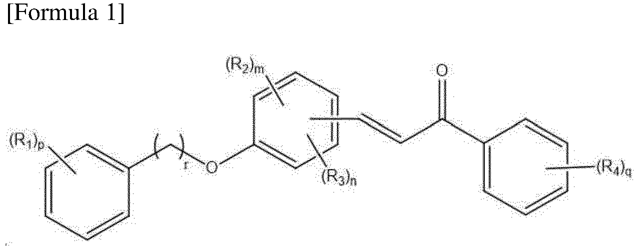 wherein:
p and q are each independently an integer ranging from 1 to 5;
r is an integer ranging from 1 to 4;
m and n are each independently an integer ranging from 1 to 3, provided that m + n is not greater than 4;
R₁ is a carbamoyl group (-C( = O)(NH2)) or a halogen, wherein when R₁ is present in a plural number, they are the same or different from each other;
R2 and R3 are each independently a C₁-C₆ alkoxy group or a halogen, wherein when each of R2 and R3 is present in a plural number, they are the same or different from each other; and
R4 comprises any one selected from the group consisting of a C1-C6 alkoxy group, an amine group, a carbamoyl group, and a hydroxyl group (-OH), wherein when R4 is present in a plural number, they are the same or different from each other.

7. A pharmaceutical composition for preventing or treating a histone acetyltransferase p300-associated disease, comprising, as an active ingredient, a compound selected from a compound represented by the following Formula 1, and a pharmaceutically acceptable salt, an optical isomer, a hydrate, and a solvate thereof: 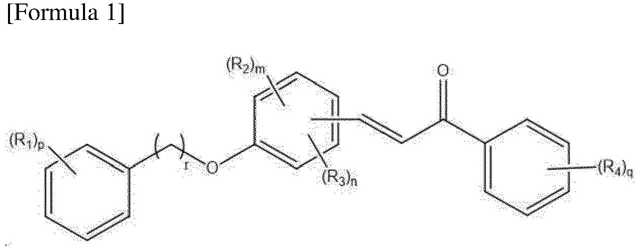 wherein:
p and q are each independently an integer ranging from 1 to 5;
r is an integer ranging from 1 to 4;
m and n are each independently an integer ranging from 1 to 3, provided that m + n is not greater than 4;
R₁ is a carbamoyl group (-C( = O)(NH2)) or a halogen, wherein when R₁ is present in a plural number, they are the same or different from each other;
R2 and R3 are each independently a C1-C6 alkoxy group or a halogen, wherein when each of R2 and R3 is present in a plural number, they are the same or different from each other; and
R4 comprises any one selected from the group consisting of a C1-C6 alkoxy group, an amine group (-NH2), a carbamoyl group, and a hydroxyl group (-OH), wherein when R4 is present in a plural number, they are the same or different from each other.

8. The pharmaceutical composition of claim 7, wherein the histone acetyltransferase p300-associated disease is fibrosis.

9. The pharmaceutical composition of claim 8, wherein the fibrosis comprises one or more selected from the group consisting of pulmonary fibrosis, uterine myoma, myelofibrosis, liver fibrosis, heart fibrosis, multiple sclerosis, kidney fibrosis, cystic fibrosis, neutropenia, skeletal muscle fibrosis, scleroderma, dermatomyositis, mediastinal fibrosis, and splenic fibrosis caused by sickle-cell anemia.

10. The pharmaceutical composition of claim 9, wherein the pulmonary fibrosis comprises one or more selected from the group consisting of idiopathic pulmonary fibrosis, nonspecific interstitial pneumonia, acute interstitial pneumonia, cryptogenic organizing pneumonia, a respiratory bronchiolitis-associated interstitial lung disease, desquamative interstitial pneumonia, lymphoid interstitial pneumonia, interstitial pulmonary fibrosis, and diffuse pulmonary fibrosis.

11. A food composition for preventing or ameliorating a histone acetyltransferase p300-associated disease, comprising, as an active ingredient, a compound selected from a compound represented by the following Formula 1, and a pharmaceutically acceptable salt, an optical isomer, a hydrate, and a solvate thereof: 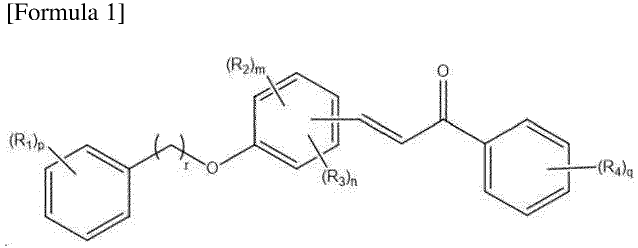 wherein:
p and q are each independently an integer ranging from 1 to 5;
r is an integer ranging from 1 to 4;
m and n are each independently an integer ranging from 1 to 3, provided that m + n is not greater than 4;
R₁ is a carbamoyl group (-C( = O)(NH2)) or a halogen, wherein when R₁ is present in a plural number, they are the same or different from each other;
R2 and R3 are each independently a C1-C6 alkoxy group or a halogen, wherein when each of R2 and R3 is present in a plural number, they are the same or different from each other; and
R4 comprises any one selected from the group consisting of a C1-C6 alkoxy group, an amine group (-NH2), a carbamoyl group, and a hydroxyl group (-OH), wherein when R4 is present in a plural number, they are the same or different from each other.

12. A cosmetic composition for preventing or ameliorating a histone acetyltransferase p300-associated disease, comprising, as an active ingredient, a compound selected from a compound represented by the following Formula 1, and a pharmaceutically acceptable salt, an optical isomer, a hydrate, and a solvate thereof: 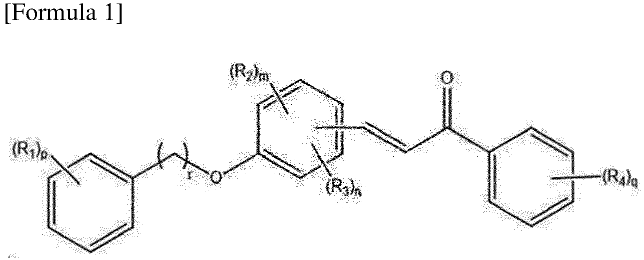 wherein:
p and q are each independently an integer ranging from 1 to 5;
r is an integer ranging from 1 to 4;
m and n are each independently an integer ranging from 1 to 3, provided that m + n is not greater than 4;
R₁ is a carbamoyl group (-C( = O)(NH2)) or a halogen, wherein when R₁ is present in a plural number, they are the same or different from each other;
R2 and R3 are each independently a C₁-C₆ alkoxy group or a halogen, wherein when each of R2 and R3 is present in a plural number, they are the same or different from each other; and
R4 comprises any one selected from the group consisting of a C1-C6 alkoxy group, an amine group (-NH2), a carbamoyl group, and a hydroxyl group (-OH), wherein when R4 is present in a plural number, they are the same or different from each other.

13. A method of preventing or treating a histone acetyltransferase p300-associated disease, comprising:
administering a compound selected from a compound represented by the following Formula 1, and a pharmaceutically acceptable salt, an optical isomer, a hydrate, and a solvate thereof to a target subject: 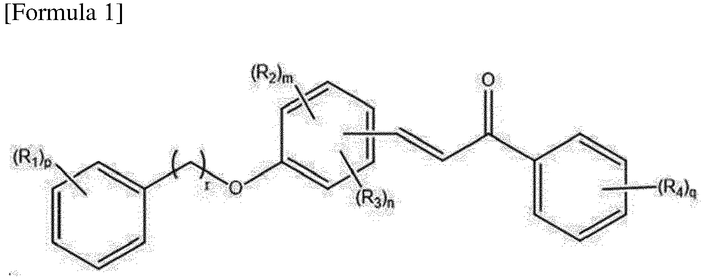 wherein:
p and q are each independently an integer ranging from 1 to 5;
r is an integer ranging from 1 to 4;
m and n are each independently an integer ranging from 1 to 3, provided that m + n is not greater than 4;
R₁ is a carbamoyl group (-C( = O)(NH2)) or a halogen, wherein when R₁ is present in a plural number, they are the same or different from each other;
R2 and R3 are each independently a C1-C6 alkoxy group or a halogen, wherein when each of R2 and R3 is present in a plural number, they are the same or different from each other; and
R4 comprises any one selected from the group consisting of a C1-C6 alkoxy group, an amine group (-NH2), a carbamoyl group, and a hydroxyl group (-OH), wherein when R4 is present in a plural number, they are the same or different from each other.

14. The method of claim 13, wherein the histone acetyltransferase p300-associated disease is fibrosis.
